# EUROPEAN PATENT SPECIFICATION

(11) **EP 3 031 496 B1**
(45) Date of publication and mention of the grant of the patent: **31.01.2018**
(21) Application number: 15198500.9
(22) Date of filing: 08.12.2015
(51) Int. Cl.: A61Q 5/02, A61Q 13/00, A61K 8/81, A61Q 19/10, A61K 8/90, A61K 8/11

(54) **RINSE-OFF COSMETIC COMPOSITIONS**
ABSPÜLBARE KOSMETISCHE ZUSAMMENSETZUNGEN
COMPOSITIONS COSMÉTIQUES DE RINÇAGE

(30) Priority: 08.12.2014 EP 14306975
(43) Date of publication of application: 15.06.2016
(73) Proprietor: Takasago International Corporation, Tokyo 144-8721 (JP)
(72) Inventor: WARR, Jonathan, 75017 PARIS (FR); FRASER, Stuart, Little Neston, Cheshire CH64 4DH (GB); CHOQUET, Emeline, 75017 PARIS (FR); LYLE, Ian, 75017 PARIS (FR)
(74) Representative: Nevant, Marc

(56) References cited:
- WO-A1-2012/126786
- WO-A2-01/74311
- WO-A2-2014/011860
- KR-A- 20080 072 422
- US-A1- 2012 276 175

## Description

### Field of the Invention

The present disclosure discloses a liquid rinse-off cosmetic composition containing an encapsulated perfume and a manufacturing method thereof.

### Background

Microencapsulation represents a common solution to protect (e.g. upon storage) and control the delivery of hydrophobic materials such as fragrances. Perfumes are expensive ingredients in cosmetic compositions and both the consumers and manufacturers would prefer that perfumes could be deposited to a great extent and enjoyed for a long time after application of the composition. Unfortunately, existing solutions to obtain these goals are not always satisfactory. Another technical aspect that may be important to consider when formulating perfume-containing, liquid rinse-off cosmetic compositions is the stability of the composition. In particular, the perfume-containing microcapsules are dispersed within the composition. It is considered to be commercially preferable and an advantage to properly deliver the perfume that the microcapsules remain dispersed (i.e. suspended) and e.g. do not irreversibly float or settle upon storage. Solutions exist to minimize separation within the dispersion such as the use of suspending agents. However, the current solutions are not always satisfactory and may bring an unexpected negative effect on microcapsule performances and perfume deposition. The present disclosure discloses a perfume-containing, liquid rinse-off cosmetic composition which is endowed with optimized delivery of perfume to e.g. hair and skin upon application of the composition. The composition may also present improved stability.

### Summary of the Invention

The instant disclosure discloses aspects such as a liquid rinse-off cosmetic composition containing a plurality of perfume-containing, formaldehyde-free microcapsules and the use of a specific ingredient to provide suspending properties appropriate to maintain the composition stable but without affecting the performances of the microcapsules. In particular, the present disclosure discloses the following points:
1. A rinse-off cosmetic composition comprising an aqueous carrier and a plurality of perfume-containing, formaldehyde-free microcapsules dispersed in the composition, wherein the composition is liquid, the composition comprises a surfactant and a suspending agent selected from the group consisting of a hydrophobically modified copolymer, a hydrophobically modified crosspolymer, a non-hydrophobically modified crosspolymer, and mixtures thereof, and wherein the microcapsules have an average particle size as median volume particle size D(v;0.5) value comprised between about 5 and 30 microns.
2. The composition according to point 1, wherein the surfactant is present in an amount comprised between about 5% and about 45% by weight over the weight of the composition.
3. The composition according to points 1 or 2, wherein the surfactant is selected from the group consisting of:
   (S1) an alkyl benzene sulfonate having formula (i) R-Ar-SO₃M wherein R is a C₃-C₂₄ alkyl group, Ar is a phenyl group, and M is a cosmetically acceptable cation;
   (S2) an alkyl sulfate having formula (ii) R-OSO₃M, wherein R and M are each independently as defined as above;
   (S3) an alkyl ether sulfate having formula (iii) R-O(C₂H₄O)ₓSO₃M, wherein R and M are each independently as defined above and x is an integer comprised between 1 and 10;
   (S4) an olefin sulfonate having formula (iv) L-SO₃M wherein L is a C₁₀-C₂₄ alkenyl group and M is as defined above;
   (S5) a sulfosuccinate;
   (S6) a surfactant selected from the group consisting of cocoamphoacetate, cocoamphodiacetate, lauroamphoacetate, lauroamphodiacetate, and mixtures thereof; and
   (S7) mixtures thereof.
4. The composition according to any one of points 1 to 3, wherein the suspending agent is present in an amount comprised about 0.01 and about 5.0 wt.% over the weight of the composition.
5. The composition according to any one of points 1 to 4, wherein the suspending agent includes at least a hydrophobically modified crosspolymer.
6. The composition according to any one or more of points 1 to 5, which is substantially free of a polymer having INCI name Acrylates Copolymer.
7. The composition according to any one or more of points 1 to 6, further comprising a polymer deposition aid.
8. The composition according to point 7, wherein the deposition aid is a cationic one.
9. The composition according to point 8, wherein the deposition aid includes a polymer containing quaternary amine groups.
10. The composition according to any one or more of points 7 to 9, wherein the deposition aid is present in an amount comprised between about 0.01 wt.% and about 5.0 wt.% by weight over the weight of the composition.
11. The composition according to any one or more of points 7 to 10, wherein the deposition aid has an average molecular weight comprised between about 250,000 and about 3 million amu.
12. The composition according to any one or more of points 7 to 11, wherein the deposition aid includes a cationic polymer derivative of natural polysaccharide polymers.
13. The composition according to point 12, wherein the cationic polymer derivative is a polymer having a C₅-C₆ sugar backbone grafted with cationic functional moieties.
14. The composition according to any one or more of points 1 to 13, further comprising a salt.
15. The composition according to any one or more of points 1 to 14, wherein the carrier includes water in an amount such that the composition contains between about 50% and 90% by weight of water.
16. The composition according to any one or more of points 1 to 15, wherein the microcapsules have the structure of a perfume composition enclosed within a polymeric shell.
17. The composition according to point 16, wherein the microcapsules have a density higher than about 0.85 and lower than about 1.1 g/ml as measured at 20 degrees and 1 atm.
18. The composition according to point 16 or 17, wherein the microcapsules have a polymeric shell which is obtainable by any one of:
   A) condensation reactions,
   B) free radical polymerization reactions, or
   C) coacervation of pre-formed polymers followed by crosslinking of the thereby obtained coacervates by using a crosslinker.
19. The composition according to any one or more of points 1-18, having a viscosity of lower than about 40000 cPs and greater than about 500 cPs when measured at 20 degrees and 1 atm by using a Brookfield viscometer at 20 or lower rpm.
20. Use of an ingredient selected from the group consisting of a hydrophobically modified copolymer, a hydrophobically modified crosspolymer, a non-hydrophobically modified crosspolymer, and mixtures thereof, as suspending agent in a liquid rinse-off cosmetic composition, said composition comprising an aqueous carrier and a plurality of perfume-containing, formaldehyde-free microcapsules dispersed therein, wherein the microcapsules have an average particle size as median volume particle size D(v;0.5) value comprised between about 5 and 30 microns.

### Detailed Description

Several ingredients discussed below in connection with the composition presently disclosed are commercially available in admixture with carriers, e.g. as solutions or dispersions wherein active ingredients are admixed with (typically water-based) solvent or dispersing carriers. Unless otherwise indicated, amounts of ingredients in the present composition are to be intended to refer to the amount of the "active ingredient", i.e. amounts of e.g. a polymer *per se,* rather than as amounts of the active ingredient-containing, commercial product.

Unless otherwise indicated, (meth)acrylate and (meth)acrylic mean methacrylate and/or acrylate and methacrylic and/or acrylic, respectively. Unless otherwise indicated, ingredients of the presently disclosed composition are cosmetically acceptable.

In one aspect, the present disclosure discloses a rinse-off cosmetic composition as defined in the appended claims, wherein the microcapsules have an average particle size as median volume particle size D(v;0.5) value comprised between about 5 and 30, for example between about 5 and 25, such as between about 5 and 20 microns or between 7.5 and 15 microns or between 10 and 15 microns.

The preferred technique used in the present disclosure to measure the microcapsule average particle size is light scattering using for example a Horiba® or a Malvern® Laser scattering particle Size Distribution analyzer or an equivalent instrument working on the principle of low angle laser light scattering (LALLS). The general guidelines set out in ISO 13320 "Particle Size Analysis - Laser Diffraction Methods" (2009 edition) may be followed. In practice, the average particle size of microcapsules may be calculated by subjecting a water dispersion of microcapsules to be analysed to the ISO 13320 standardized procedure. Alternatively, measurement can be performed with a laser diffraction/scattering particle size distribution analyzer (for example the one having trade name: LA-950V2, manufactured by Horiba, Ltd.) according to the following protocol: the dispersant is deionized water purified to 18 MΩ; several droplets of the capsule dispersion are poured into the flow cell unit of the analyzer until an acceptable level of laser light obscuration is achieved and triplicate measurements are then immediately performed. For the calculation of the particle size measurement, the following refractive indexes may be used: 1.33 for the water dispersant, 1.47 for the fragrances and e.g. 1.47 for the microcapsule shells in the absence of measured values. These refractive indexes are considered to be acceptable experimental approximations in the absence of already measured indexes for a given water dispersant, fragrance or shell. The median capsule diameter is measured as a particle size of 50% frequency (median size) on a volumetric basis.

Unless otherwise indicated, rinse-off is defined as per the Regulation (EC) No. 1223/2009 of the European Parliament and of the Council of 30 November 2009 on cosmetic products (recast), i.e. a cosmetic product or composition is a rinse-off one when it is intended to be removed after application on skin, hair or mucous membranes of a human subject. The rinse-off cosmetic composition presently disclosed may be a rinse-off, cosmetic cleansing composition, for example a rinse-off, cosmetic, personal care, cleansing composition. Examples of the rinse-off cosmetic composition (hereinafter also "the composition") are a shower gel composition, a liquid soap, a body wash and a shampoo. The composition is not edible or comestible, like toothpastes.

Advantageously, "liquid" means that the composition has a viscosity lower than about 40000 cPs, such as lower than about 30000 or lower than about 20000 cPs or lower than 15000 cPs, and greater than for example about 500 cPs, or greater than about 1000cPs or greater than about 2000 cPs, or greater than 5000 cPs or greater than 6500 cPs when measured at 20 degrees and 1 atm by using a Brookfield viscometer at for example 20 or lower rpm. The choice of an appropriate spindle can be made by a skilled man based on e.g. information provided by viscometer manufacturers and the expected viscosity range of the composition to be tested.

Every numerical range given throughout this disclosure (e.g. "between about 1 and about 10") will include the minimum and maximum end values of that range (i.e. 1, respectively 10, in the example given above).

The composition comprises a surfactant, such as a detersive surfactant.

For example, the surfactant may be present in an amount comprised between about 5% and about 45%, for example between about 7.5% and about 30%, such as from about 10% to 25%, or also between about 12% and 20% by weight over the weight of the composition.

The surfactant may be selected from the group consisting of:
(S1) an alkyl benzene sulfonate having formula (i) R-Ar-SO₃M wherein R is a C₃-C₂₄ alkyl group, Ar is a phenyl group, and M is a cosmetically acceptable cation, for example selected from the group consisting of ammonium ion, a charged alkanolamine ion (such as a cationic triethanolamine), an alkali metal ion (such as sodium or potassium ions), and mixtures thereof;
(S2) an alkyl sulfate having formula (ii) R-OSO₃M, wherein R and M are each defined as above;
(S3) an alkyl ether sulfate having formula (iii) R-O(C₂H₄O)ₓSO₃M, wherein R and M are each independently as defined above and x is an integer comprised between 1 and 10;
(S4) an olefin sulfonate having formula (iv) L-SO₃M wherein L is a C₁₀-C₂₄ alkenyl group and M is as defined above;
(S5) a sulfosuccinate;
(S6) a surfactant selected from the group consisting of cocoamphoacetate, cocoamphodiacetate, lauroamphoacetate, lauroamphodiacetate, and mixtures thereof; and
(S7) mixtures thereof.

For example, the surfactant may be selected from the group consisting of: a surfactant (S1), a surfactant (S2), a surfactant (S3), and mixtures thereof. For example, the surfactant may include at least a surfactant (S4). For example, the surfactant may include at least a surfactant (S5). For example, the surfactant may include at least a surfactant (S6).

A preferred alkyl ether sulfate is obtainable by condensing a monohydric alcohol having from about 8 to about 24 carbon atoms, such as an alcohol derived from petrochemical feed stocks or natural oils e.g. coconut oil, palm kernel oil, or tallow, with ethylene oxide, for example with from about 1 to about 10, such as from about 1 to about 5, for example from about 1 to about 3 moles ethylene oxide per mole of alcohol, sulfating the condensation product and neutralizing the sulfated product. The alcohol may be lauryl alcohol or a straight chain C₈-C₂₄ alcohol derived from coconut or palm kernel oil.

Examples of sulfosuccinate are disodium N-octadecylsulfosuccinate, disodium lauryl sulfosuccinate, diammonium lauryl sulfosuccinate, tetras odium N-(1,2-dicarboxyethyl)-N-octadecylsulfosuccinate, diamyl ester of sodium sulfosuccinic acid, dihexyl ester of sodium sulfosuccinic acid, dioctyl esters of sodium sulfosuccinic acid, and mixtures thereof.

Examples of surfactants are ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth sulfate, ammonium cocoyl sulfate, ammonium lauroyl sulfate, sodium cocoyl sulfate, sodium lauroyl sulfate, potassium cocoyl sulfate, potassium lauryl sulfate, triethanolamine lauryl sulfate, triethanolamine lauryl sulfate, monoethanolamine cocoyl sulfate, monoethanolamine lauryl sulfate, sodium tridecyl benzene sulfonate, sodium dodecyl benzene sulfonate, and mixtures thereof.

The surfactant may be a combination of a primary surfactant, which is any one of surfactants (S1) to (S7) as defined above, and a secondary surfactant, which is different from the primary one.

The secondary surfactant may be selected from cationic, anionic, non-ionic, zwitterionic, amphoteric surfactants and mixtures thereof. However, it is preferable that composition be substantially free of cationic surfactants. For example, the composition may contain less than 5%, such as 0% by weight over the weight of the composition of cationic surfactants. Cationic surfactants may be cetyl pyridinium chloride and/or surfactants having formula X⁻ N⁺R₁R₂R₃R₄ wherein
- one, two or three of R₁, R₂, R₃, or R₄ are each independently a C₈-C₂₄ alkyl group, and the other groups are each independently a C₁-C₇ alkyl or benzyl group, wherein, in an independent manner, each alkyl group is optionally substituted with an hydroxyl (-OH) group and optionally contains an ester (-COO-) and/or ether (-O-) spacer; and
- X⁻ is an anion selected from a halide, acetate, citrate, lactate, glycolate, phosphate, nitrate, sulfate and methosulfate.

The cationic surfactant may be a mono-C₈-C₂₄alkyl trimethyl ammonium salt. Examples include lauryl trimethylammonium chloride (e.g. Arquad C35 ex Akzo Nobel), cetyl trimethylammonium chloride (e.g. Genamin CTAC ex Clariant), behenyl trimethylammonium chloride (e.g. Genamin KDMP ex Clariant), cocodimethyl benzyl ammonium chloride (e.g. Arquad DMCB-80 ex Akzo Nobel), distearyl dimethylammonium chloride, also known as quaternium 5 using INCI nomenclature (e.g. Ethoquad C-12, Ethoquad CB12 ex Akzo Nobel), PEG-5 cocomonium methosulfate (e.g. Rewoquat CPEM ex Evonik).

In case of combination of a primary and a secondary, distinct surfactant, it is preferable that the ratio between the primary to secondary surfactant is comprised between 20:1 and 1:1, such as between 10:1 and 2:1.

An example of secondary surfactant may be an anionic surfactant obtainable by esterifying a fatty acid, such as a fatty acid derived from coconut oil or palm kernel oil, with isethionic acid and neutralizing the esterification product for example with sodium hydroxide.

Another example of secondary surfactant may be a sodium or potassium salt of fatty acid amides of methyl tauride in which the fatty acids may be derived from coconut oil or palm kernel oil.

Another example of secondary surfactant may be selected from the group consisting of sodium lauryl sarcosinate, sodium lauroyl sarcosinate, lauryl sarcosine, cocoyl sarcosine, sodium cocoyl isethionate, and mixtures thereof.

A suitable example of secondary surfactant may have an HLB of 7 or more and comprise one or more polyethyleneoxide chains, each chain having independently on average at least about 5, preferably from about 10 to 20, ethylene oxide units.

Another example of secondary surfactant may be selected from the group consisting of polyoxyethylene alkyl ethers, polyethyleneglycol fatty acid esters, polyoxyethylene castor oil, polyoxyethylene hydrogenated castor oil, polyoxyethylene fatty amides and their monoethanolamine and diethanolamine derivatives, and polyethoxylated fatty amines, and mixtures thereof.

A particular example of secondary surfactant is steareth-10 and/or steareth-15. Alternatively, a suitable example of secondary surfactant may have an HLB of 7 or more and does not contain polyethyleneoxide chains.

Another example of secondary surfactant may be a soap. Unless otherwise indicated, a soap is presently defined as a monovalent salt of a monocarboxylic alkanoic or alkenoic acid having a C₈-C₂₄ carbon chain length. The monovalent cation may be selected from the group consisting of sodium, potassium, ammonium, mono-, di-, tri-alkanolammonium ions, and mixtures thereof. Preferred sources of the alkyl chain are plant or animal oils and fats or petrochemically derived fatty acids. Example sources of plant and animal aliphatic monocarboxylic acids are coconut oil, palm oil, palm kernel oil, soybean oil, rapeseed oil, fish oil or animal tallow. These oil and fats may be hydrogenated to reduce the amount of alkenoic acids before use.

Another example of secondary surfactant may be selected from the group consisting of polyglycerolated fatty acids, polyglycerolated fatty amides, polyglycerolated alpha-diols, polyglycerolated alcohols, alkyl polyglucosides, sugar esters, and mixtures thereof, preferably, alkyl polyglucosides, sugar esters, polyglyceryl fatty acid esters, alkyl polyglyceryl ethers, and mixtures thereof.

Another example of secondary surfactant may be a secondary or tertiary amine, wherein the nitrogen atom is substituted with at least one C₈-C₁₈ aliphatic straight or branched hydrocarbon chain containing an anionic group or a group reasonably expected to become anionic when in water at a pH comprised between 3 and 9, such as a carboxy, sulfonate, sulfate, phosphate, or phosphonate group.

Another example of secondary surfactant may be a aliphatic quaternary ammonium, phosphonium, and/or sulfonium compound, wherein the nitrogen, phosphorous and sulfur atoms are substituted with at least one C₈-C₁₈ aliphatic straight or branched hydrocarbon chain containing an anionic group or a group reasonably expected to become anionic when in water at a pH comprised between 3 and 9, such as a carboxy, sulfonate, sulfate, phosphate or phosphonate group.

A particular example of secondary surfactant is a betaine, such as cocoamidopropyl betaine.

Suitable examples of secondary surfactant may be any combinations of the various examples disclosed above. For the purposes of the present disclosure, any surfactant not listed as either a primary or secondary surfactant above is to be considered as a secondary surfactant.

The present inventors found that certain ingredients (usually polymers) conventionally used as suspending agents for perfume-containing microcapsules in liquid rinse-off cosmetic compositions can affect the performances of the capsules, notably in terms of perfume deposition, even when a deposition aid is included in the formulation. This undesired phenomenon may become particularly strong for high concentrations. By contrast, the use of a suspending agent selected from the group consisting of a hydrophobically modified copolymer, a hydrophobically modified crosspolymer, a non-hydrophobically modified crosspolymer, and mixtures thereof, was found to provide acceptable stability to the composition while not affecting the performances of the perfume-containing capsules, even at high concentrations.

Accordingly, the composition comprises a suspending agent selected from the group consisting of a hydrophobically modified copolymer, a hydrophobically modified crosspolymer, a non-hydrophobically modified crosspolymer, and mixtures thereof.

The suspending agent as presently defined may be present in an amount comprised about 0.01 and about 5.0, such as between about 0.025 and about 2.0 or between about 0.05 and about 1, such as between about 0.1 and about 1.0 or between about 0.2 and about 1.0 wt.% for example at 0.6 wt.%, over the weight of the composition.

Preferably the suspending agent includes at least a hydrophobically modified crosspolymer. For example, the suspending agent comprises, such as consists of, a hydrophobically modified crosspolymer. Although it is not intended to be bound by any theory, it has been found that the presence of this crosspolymer generally provides for the best compromise between perfume performances and stability of the composition. The suspending agent may include, such as consist of, a combination of a hydrophobically modified crosspolymer and a non-hydrophobically modified crosspolymer. The suspending agent may include, such as consist of, a combination of a hydrophobically modified crosspolymer and a hydrophobically modified copolymer.

The hydrophobically modified crosspolymer may be a (meth)acrylate-containing crosspolymer. Commercially available products containing hydrophobically modified crosspolymers are Ultrez® 20, Ultrez® 21, Pemulen® TR-1, Pemulen® TR-2, Carbopol® 1342 Carbopol® 1382, Carbopol® ETD2020 all from Lubrizol, Aculyn® 88, Aculyn® 38 from Dow Chemical, SepiMAX® ZEN from Seppic, Tego® Carbomer 841 and Tego® Carbomer 341 both from Evonik.

The (meth)acrylate-containing crosspolymer may be a crosslinked copolymer obtainable by polymerizing a monomer mixture, said mixture containing at least one hydrophobically modified (meth)acrylate and a crosslinker.

Suitable hydrophobically modified (meth)acrylates may be selected from the group consisting of a higher alkyl (meth)acrylate, for example a C₈-C₃₀ alkyl (meth)acrylate, an ethoxylated higher alkyl (meth)acrylate, for example steareth-20 (meth)acrylate or laureth-4 (meth)acrylate, and mixtures thereof.

A suitable crosslinkers may be a monomer which has at least two vinyl groups capable of reacting by free radical polymerization, such as a monomer selected from the group consisting of an allyl ether of pentaerythritol, an allyl derivative (such as ether or ester) of trimethylolpropane, propenyl sucrose ether, propenyl 2,2-dihydroxymethyl-1,3-propanediol, and mixtures thereof.

The (meth)acrylate-containing crosspolymer may be obtainable by polymerizing:
∘ a monomer selected from the group consisting of (meth)acrylic acid, lower alkyl (meth)acrylate, such as C₁-C₆ alkyl (meth)acrylate, alkali metal salts of (meth)acrylic acid, such as sodium or potassium (meth)acrylic acid salts, and mixtures thereof,
∘ a hydrophobically modified (meth)acrylate, for example as defined above, and
∘ a crosslinker, for example as defined above.

For example, the (meth)acrylate-containing crosspolymer may be obtainable by polymerizing:
∘ a monomer selected from the group consisting of acrylic acid, lower alkyl acrylate, such as C₁-C₆ alkyl acrylate, alkali metal salts of acrylic acid, such as sodium or potassium acrylic acid salts, and mixtures thereof,
∘ a monomer selected from the group consisting of methacrylic acid, lower alkyl methacrylate, such as C₁-C₆ alkyl methacrylate, alkali metal salts of methacrylic acid, such as sodium or potassium methacrylic acid salts, and mixtures thereof,
∘ a hydrophobically modified (meth)acrylate, for example as defined above, and
∘ a crosslinker, for example as defined above.

The hydrophobically modified crosspolymer may be selected from the group consisting of (INCI names)
Acrylates/C10-30Alkyl Acrylate Crosspolymer;
Acrylates/C12-13 Alkyl Methacrylates/Methoxyethyl Acrylate Crosspolymer; Acrylates/Ethylhexyl Acrylate Crosspolymer;
Acrylates/Ethylhexyl Acrylate/Glycidyl Methacrylate Crosspolymer;
Acrylates/Steareth-20 Methacrylate Crosspolymer;
Acrylates/Vinyl Isodecanoate Crosspolymer;
Acrylates/Vinyl Neodecanoate Crosspolymer;
C8-22 Alkyl Acrylates/Methacrylic Acid Crosspolymer;
Lauryl Methacrylate/Glycol Dimethacrylate Crosspolymer;
Lauryl Methacrylate/Sodium Methacrylate Crosspolymer;
Potassium Acrylates/C10-30 Alkyl Acrylate Crosspolymer;
Sodium Acrylates/C10-30 Alkyl Acrylate Crosspolymer;
Sodium Acrylates/Vinyl Isodecanoate Crosspolymer;
Stearyl/Lauryl Methacrylate Crosspolymer;
Polyacrylate Crosspolymer-6;
and mixtures thereof.

It may be advantageous that the hydrophobically modified crosspolymer is selected from the group consisting of (INCI names) Acrylates/C10-30Alkyl Acrylate Crosspolymer, Acrylates/Steareth-20 Methacrylate Crosspolymer, Polyacrylate Crosspolymer-6, and mixtures thereof.

The hydrophobically modified copolymer may be a (meth)acrylate-containing copolymer. Examples of commercially available product containing hydrophobically modified copolymers are Aculyn® 22 and Aculyn® 28 from Dow Chemical.

The (meth)acrylate-containing copolymer may be a non-crosslinked copolymer obtainable by polymerizing a monomer mixture, said mixture containing at least one hydrophobically modified (meth)acrylate, for example as defined above.

The (meth)acrylate-containing copolymer may be obtainable by polymerizing:
∘ a monomer selected from the group consisting of (meth)acrylic acid, lower alkyl (meth)acrylate, such as C₁-C₆ alkyl (meth)acrylate, alkali metal salts of (meth)acrylic acid, such as sodium or potassium (meth)acrylic acid salts, and mixtures thereof, and
∘ a hydrophobically modified (meth)acrylate, for example as defined above.

For example, the (meth)acrylate-containing copolymer may be obtainable by polymerizing:
∘ a monomer selected from the group consisting of acrylic acid, lower alkyl acrylate, such as C₁-C₆ alkyl acrylate, alkali metal salts of acrylic acid, such as sodium or potassium acrylic acid salts, and mixtures thereof,
∘ a monomer selected from the group consisting of methacrylic acid, lower alkyl methacrylate, such as C₁-C₆ alkyl methacrylate, alkali metal salts of methacrylic acid, such as sodium or potassium methacrylic acid salts, and mixtures thereof, and
∘ a hydrophobically modified (meth)acrylate, for example as defined above.

A particularly suitable (meth)acrylate-containing copolymer has INCI name Acrylates/Steareth-20 Methacrylate Copolymer.

The non-hydrophobically modified crosspolymer may be a crosslinked polyacrylic acid polymer, for example a high molecular weight, non-linear, polyacrylic acid crosslinked with polyalkenyl polyether, such as a crosspolymer having INCI name "Carbomer", i.e. a poly(acrylic acid) crosslinked with 2,2-bis(hydroxymethyl)propane-1,3-diol 2-propenyl ether. Examples of commercially available products containing non-hydrophobically modified crosspolymer are Carbopol® 934, 940, 941, 980, 981, Carbopol® silk 100, Carbopol® ETD 2050, Carbopol® Ultrez 10 and Carbopol® Ultrez 30, all from Lubrizol.

If desired, a suspending agent as presently defined may include further ingredients commonly known to have suspending properties, such an additional suspending ingredient selected from the group consisting of cellulose derivatives, modified cellulose polymers, starch derived and modified polymers, other natural polymeric gums and derivatives, and mixtures thereof. For example, the further suspending agents may be selected from the group consisting of methyl cellulose, ethyl cellulose, hydroxyethyl cellulose, hydroxypropyl methyl cellulose, nitro cellulose, sodium cellulose sulfate, sodium carboxymethyl cellulose, crystalline cellulose, cellulose powder, xanthan gum, arabia gum, tragacanth, galactan, carob gum, karaya gum, carrageenan, pectin, agar, starch (rice, corn, potato, wheat), algae colloids, microbiological polymers such as dextran, succinoglucan, pulleran, starch-based polymers such as carboxymethyl starch, methylhydroxypropyl starch, alginic acid-based polymers such as sodium alginate, and alginic acid propylene glycol esters, nonoxynyl hydroxyethylcellulose, ethylene oxide and/or propylene oxide based polymers, and mixtures thereof.

Alternatively, the composition may be substantially free of (such as free of) suspending agents other than a hydrophobically modified copolymer, a hydrophobically modified crosspolymer, a non-hydrophobically modified crosspolymer, and mixtures thereof. In particular, it is preferable that the composition is substantially free, such as free, of at least a polymer having INCI name "Acrylates Copolymer", i.e. a copolymer obtainable by polymerizing acrylic acid, methacrylic acid, ethyl acrylate and methyl methacrylate. A commercially available product containing INCI Acrylates Copolymer is Aqua SF1 from Lubrizol. Polymers having INCI name "Acrylates Copolymer", despite being common suspending agents in rinse-off cosmetic compositions, were found to affect the performances of the capsules, notably in terms of perfume deposition.

Finally, it is noted that a skilled man would have no hesitations as to whether a given suspending agent, is hydrophobically modified or not as well as whether it is a copolymer or crosspolymer. In effect, for any commercially named polymer suitable to be used in a Cosmetic product there is an INCI name. For example Aqua SF1 has INCI name Acrylates Copolymer. The fact that it is called a copolymer indicates that it is a copolymer and not a crosspolymer. The European Commission maintains a Cosmetic Ingredients and Substances Database (known as the "Coslng" database and well-known to any skilled man working in the cosmetic field) with further details for all INCI polymers. The INCI name of the polymer can be entered into this database to search e.g. for the Chemical/IUPAC name of the polymer. In the case of Aqua SF1, "Acrylates Copolymer" has the Chemical/IUPAC name: 2-propenoic acid, 2-methyl-, polymer with ethyl 2-propenoate and methyl 2-methyl-2-propenoate. Based on the chemical nature its constituting monomers, a given polymer can be readily recognized as being hydrophobically modified or not.

The composition advantageously comprises a polymer deposition aid, such as a cationic polymer deposition aid.

The deposition aid may be present in an amount comprised between about 0.01% to about 5%, for example between about 0.05% to about 3%, such as from about 0.075% to about 2.0%, for example from about 0.1% to about 1.0% by weight over the weight of the composition.

A polymer deposition aid may be cationic for example because:
i) it is a polymer containing a net (i.e. permanently) positively-charged atom(s) or chemical group(s) covalently linked thereto, such as quaternary amine groups, and/or
ii) it is a polymer containing atom/s or chemical group(s) covalently linked thereto and that are reasonably anticipated to become cationic when put in water at a pH comprised between about 3 and 9, such as between 4 and 8, for example primary, secondary, tertiary amine groups.

Preferably, the polymer deposition aid herein disclosed includes, such as consists of a polymer containing a net (i.e. permanently) positively-charged atom(s) or chemical group(s) covalently linked thereto, such as quaternary amine groups.

Unless otherwise indicated, "reasonably anticipated" means that a skilled reader based on his knowledge would expect a given physical or chemical composition or characteristic (in this case the formation of a cationic charge on the deposition aid) to occur, based on factors such as the nature of the precursors used to manufacture the polymer, the type of reaction, the type of manufacturing process, the products produced in the polymerization, the intended uses of the substance and associated use conditions. The formation of a cationic charge may be the result of a chemical equilibrium, i.e. a certain amount of the atoms and/or groups that are reasonably anticipated to become cationic when put in water at a given pH may remain uncharged based e.g. on the acid/basic characteristics of those atoms and/or groups *per se* or when considered in the context of the polymer deposition aid.

The polymer deposition aid, such as a cationic one, may have an average molecular weight comprised between about 10,000 and 10 million atomic mass units (amu; 1 amu is equivalent to 1 g/mol), for example between about 100,000 and about 5 million amu, such as between about 250,000 and about 3 million amu. A polymer deposition aid having high molecular weights such as greater than 250,000, greater than 500,000 or greater than 1 million amu and lower than e.g. 3 million amu are preferred. A polymer deposition aid with lower molecular weights (such as lower than 10,000 amu, for example greater than 1,000 or greater than 2,500 amu) may be obtainable by hydrolysis of a starting polymer deposition aid. Subsequent derivatization with a cationic functional moiety is possible.

A cationic polymer deposition aid presently disclosed may have a cationic charge density of at least 0.1 meq/g, such as greater than 0.4 meq/g, for example greater than 0.8meq/g and lower than 4 meq/g, such as lower than 3, for example lower than 2 meq/g, for example a charge density comprised between about 0.8 and 1.5 milliequivalents/gram (meq/g), at a pH comprised between about 3 and 9, such as between 4 and 8. Unless otherwise indicated, the cationic charge density of a given deposition aid is presently defined as the amount of cationic charge per gram of given polymer deposition aid. The charge density can be measured using conductometric analysis at the pH specified. High charge densities, such as greater than 0.8, for example greater than 1.0 meq/g, are preferred, even more preferred if combined with high molecular weights as defined above. Charge densities of some specific deposition aids presently disclosed are listed in Table 1:

**Table 1**

| **Commercial name** | **Chemical name (chemical or INCI)** | **Supplier** | **Charge density class** | **Charge density value (provided by supplier)** |
|---|---|---|---|---|
| Jaguar C17 | Guar hydroxypropyl trimonium chloride | Solvay | high | 1.1 meq/g |
| Jaguar C162 | Hydroxypropyl guar hydroxypropyltrimonium chloride | Solvay | medium | 0.7 meq/g |
| Jaguar C-13-S | Guar hydroxypropyl trimonium chloride | Solvay | medium | 0.8 meq/g |
| Jaguar Excel | Guar hydroxypropyl trimonium chloride | Solvay | medium | 0.7 meq/g |
| Ucare JR400 | Polyquaternium-10 | Dow | high | 1.06 meq/g |
| SoftCat polymer SX-1300H | Polyquaternium- 67 | Dow | high | 1.45 meq/g |
| Dehyquart CC7 BZ | Polyquaternium -7 | BASF | high | 1.6 meq/g |

A cationic polymer deposition aid presently disclosed may have a high molecular weight, e.g. between about 1 and 2 million amu, and high charge density, e.g. charge densities close to or in excess of 1 meq/g.

The cationic polymer deposition aid may include a counterion, for example halides (e.g., chloride, fluoride, bromide and iodide), sulfate and/or methyl sulfate.

The deposition aid may include, such as consist of, a cationic polymer derivative of natural polysaccharide polymers. This polymer (also herein referred to as "cationic polymer derivative") may be a polymer having a natural polysaccharide backbone made cationic by cationic functional moieties grafted thereon. For example, the cationic polymer derivative may be a polymer having a C₅-C₆ sugar backbone grafted with cationic functional moieties. For example, the cationic polymer derivative may be obtainable by derivatizing a polymer having a C₅-C₆ sugar backbone, e.g. by grafting cationic functional moieties thereon. Cationic functional moieties (and more broadly cationic groups) are defined as net (i.e. permanently) positively-charged atom/s or chemical group/s covalently linked to a given polymer, or atom/s or chemical group/s covalently linked to a given polymer and that are reasonably anticipated to become cationic when put in water at a pH comprised between about 3 and 9, such as between 4 and 8, for example primary, secondary, tertiary or aromatic amine groups.

Examples of the cationic polymer derivative are a cationic cellulose; a cationic hydroxyethyl cellulose; a cationic starch; a cationic hydroxyalkyl starch; a cationic polymer including arabinose monomers (the natural polymer being for example obtainable from vegetable gums); a cationic polymer obtainable by derivatizing xylose polymers (the natural polymer being present for example in materials such as wood, straw, cottonseed hulls, and corn cobs); a cationic polymer obtainable by derivatizing fucose polymers (the natural polymer being present for example as a component of cell walls in seaweed); a cationic polymer obtainable by derivatizing fructose polymers such as inulin; a cationic polymer including acid-containing sugars such as galacturonic acid and glucuronic acid; a cationic polymer including amine sugars such as galactosamine and glucosamine; a cationic polymer including 5- and/or 6-membered ring polyalcohols; a cationic polymer including galactose monomers (the natural polymer occurring for example in plant gums and mucilages); a cationic polymer including mannose monomers (the natural polymer occurring for example in plants, yeasts, and red algae); a cationic polymer including a galactomannan copolymer known as guar gum which is obtainable for example from the endosperm of the guar bean, and mixtures thereof.

A preferred example of the cationic polymer derivative is a cationic guar gum polymer wherein the polymer is obtainable by grafting a polysaccharide backbone of galactomannan units with a cationic functional moiety, such as an adduct of 2,3-epoxypropylytrimethyl ammonium chloride. An example is polymer having INCI name Guar Hydroxypropyltrimonium Chloride. Commercial examples can be found in the ESAFLOR range of polymers by Lamberti Industries, the Jaguar range from Solvay or the N-Hance range from Ashland Chemicals.

Other commercially available examples of the cationic polymer derivative are cationic hydroxyethyl celluloses (INCI name Polyquaternium-10) available in the Ucare JR and LR ranges from Amerchol and Dow; the substitution product of 2-hydroxyethylcellulose ether with both trimethyl ammonium and dodecyl dimethylammonium salts (INCI name Polyquaternium-67) and available under the SoftCat brand name from Dow; quaternary nitrogen-containing cellulose ethers e.g. as described in US3962418 and copolymers of etherified cellulose and starch e.g as described in US 3958581

The deposition aid may include, such as consist of, a cationic polymer derivative as presently defined combined with a distinct, cationic polymer. For example this latter polymer may be a synthetic polymer of a cationic vinyl monomer or a vinyl monomer reasonably anticipated to become cationic when put in water at a pH comprised between about 3 and 9, such as a vinyl monomer having protonated amine or quaternary ammonium functionalities, with at least a water soluble spacer monomer such as acrylamide, methacrylamide, alkyl and dialkyl acrylamides, alkyl and dialkyl methacrylamides, alkyl acrylate, alkyl methacrylate, vinyl caprolactone or vinyl pyrrolidone.

The synthetic polymer may for example include a monomer selected from the group consisting of dialkylaminoalkyl acrylate, dialkylaminoalkyl methacrylate, monoalkylaminoalkyl acrylate, monoalkylaminoalkyl methacrylate, trialkyl methacryloxyalkyl ammonium salt, trialkyl acryloxyalkyl ammonium salt, diallyl quaternary ammonium salts, vinyl quaternary ammonium monomers having cyclic cationic nitrogen-containing rings such as pyridinium, imidazolium, and quaternized pyrrolidone, e.g., alkyl vinyl imidazolium, alkyl vinyl pyridinium, alkyl vinyl pyrrolidone salts, and mixtures thereof. The synthetic polymer may be selected from the group consisting of:
∘ a copolymer of 1-vinyl-2-pyrrolidone and 1-vinyl-3-methylimidazolium salt (e.g., chloride salt having INCI name Polyquaternium-16);
∘ a quaternarized copolymer of 1-vinyl-2-pyrrolidone and dimethylaminoethyl methacrylate (e.g. INCI name Polyquaternium-11);
∘ a cationic diallyl quaternary ammonium-containing polymer, for example, dimethyldiallylammonium chloride homopolymer and copolymers of acrylamide and dimethyldiallylammonium chloride (INCI names as Polyquaternium-6 and Polyquaternium-7, respectively);
∘ an amphoteric copolymer of acrylic acid for example a copolymer of acrylic acid and dimethyldiallylammonium chloride (INCI name Polyquaternium-22), a terpolymer of acrylic acid with dimethyldiallylammonium chloride and acrylamide (INCI name Polyquaternium-39), and a terpolymer of acrylic acid with methacrylamidopropyl trimethylammonium chloride and methyl acrylate (INCI name Polyquaternium-47);
∘ polymethacrylamidopropyl trimonium chloride, and
∘ mixtures thereof.

Cationic polymers disclosed herein may be the sole deposition aids present in the composition. For example, the composition contains a polymer derivative, optionally combined with a synthetic cationic polymer, both as presently defined as sole deposition aids. For example, the composition may be free of cationic (polymer or non-polymer) deposition aids not containing quaternary amine groups (i.e. permanently charged groups as opposed to pH dependent primary, secondary and ternary amines).

Designing the perfume-containing capsules so that they have a density matching the one of conventional rinse-off cosmetic compositions may be an approach that helps reducing the amount of certain unsatisfactory suspending agents and/or eliminating the presence of other, particularly problematic ones. Accordingly, it may be advantageous that the microcapsules to be used in the present composition have a density higher than about 0.85 g/ml, for example higher than 0.95g/ml and lower than about 1.1 g/ml, measured as disclosed below.

The composition may further comprise a salt for example to modify (e.g. increase) the viscosity of the rinse-off cosmetic composition. The salt may be a cosmetically-acceptable organic and/or inorganic one, for example a cosmetically-acceptable inorganic salt. The salt may be combination of two or more salts as presently defined. The inorganic salt may consist of a mineral acid anion, such as chloride, nitrate, phosphate, or sulfate, and a cation which is selected from the group consisting of a metal ion from group 1 or 2 of the periodic table, preferably a sodium, potassium or magnesium ion; ammonium ion; a mono-, di- or tri-alkonium ion; and mixtures thereof. Examples of preferred inorganic salts are sodium chloride and potassium chloride. The organic salt may consist of a cation as described above and an organic anion selected from the group consisting of acetate, propionate, citrate, benzoate, sorbate and salicylate. Although the primary function of salts is to modify the viscosity of the rinse-off cosmetic composition, they may also play secondary functions (for example sodium benzoate which is also a preservative). Also, salts may be added to the composition as part of other ingredients (e.g. it is known that surfactants may contain inorganic salts such as sodium chloride).

For the sake of clarity, it is considered that irrespective of their function or source/origin, the composition may contain salts as defined above in an amount comprised between about 0% and 5%, for example between about 0.5% and 5% by weight over the weight of the rinse-off cosmetic composition. The exact amount may be adjusted by a skilled man depending on the specific composition and required product properties.

The aqueous carrier includes, such as consists of water. Preferably it contains water in an amount such that the composition contains between about 50% and 90%, such as between about 60% and 85% w/w of water. These amounts of water are typically sufficient to impart a liquid state (as presently defined) to the composition at 20 degrees and 1 atm.

The aqueous carrier may contain water in lower amounts, for example in amounts such that the composition contains e.g. lower than 50% w/w, such as between 20% and 45% w/w of water. However, in this case, the resulting composition is in the form of a concentrated composition which suitably generates a ready-to-use one upon dilution e.g. by final user.

The composition may further include a conditioning agent and/or an anti-dandruff agent. Suitable conditioning agents for use in the composition are those conditioning agents characterized generally as silicones (e.g., silicone oils, cationic silicones, silicone gums, high refractive silicones, and silicone resins), organic conditioning oils (e.g., hydrocarbon oils, polyolefins, and fatty esters) or combinations thereof. Suitable, non-limiting examples of anti-dandruff agents include: antimicrobial actives, pyridinethione salts, azoles, selenium sulfide, particulate sulfur, keratolytic acid, salicylic acid, octopirox (piroctone olamine), coal tar, and combinations thereof. In one aspect, the anti-dandruff agents typically are pyridinethione salts.

The composition may further include one or more ingredients selected from a foam booster, an opacifier, a pearlescent agent, a dye, a coloring agent, a preservative, a protein, a buffering agent and an unencapsulated perfume.

Based on the above, examples of rinse-off cosmetic compositions according to the present disclosure may be those including:
- a plurality of perfume-containing, formaldehyde-free microcapsules as presently defined, and notably having an average particle size as median volume particle size D(v;0.5) value comprised between about 5 and 30, for example between about 5 and 25, such as between about 5 and 20 microns or between 7.5 and 15 microns or between 10 and 15 microns,
- said microcapsules being dispersed in the composition,
- the compositions comprising a surfactant, preferably in an amount comprised between about 5% and about 45% by weight over the weight of the composition,
- the compositions comprising an aqueous carrier, the carrier comprising water, preferably in an amount such that the composition contains between about 50% and 90% by weight of water,
- a suspending agent as presently defined, preferably in an amount comprised between about 0.01 wt.% and about 5.0 wt.% by weight over the weight of the composition,
and wherein the composition may further include one or more of features a) to c) below, for example at least feature a), for example at least feature b), for example at least features a) and b), for example all features a) to c):
a) a polymer deposition aid, preferably in an amount comprised between about 0.01 wt.% and about 5.0 wt.% by weight over the weight of the composition;
b) a salt, preferably in an amount comprised between 0.5% and 5% by weight over the weight of the composition;
c) an ingredient selected from the group consisting of a conditioning agent, an anti-dandruff agent, a foam booster, an opacifier, a pearlescent agent, a dye, a coloring agent, a preservative, a protein, a buffering agent, an unencapsulated perfume, and mixtures thereof.

The microcapsules included in the composition presently disclosed have typically the structure of a perfume composition enclosed within a polymeric shell. Microcapsules of this type may be well-known core-shell microcapsules.

The microcapsules are formaldehyde-free. Irrespective of the chemical process used to encapsulate the perfume composition, the plurality of microcapsules is initially obtained in the form of a dispersion in water (which is the dispersing phase). This dispersion may then be further processed in order to incorporate the plurality of microcapsules into the rinse-off cosmetic composition. Whether microcapsules are formaldehyde-free as presently defined can be easily verified on that water dispersion. In particular, microcapsules are formaldehyde-free if an analysis of a sample of the dispersing phase (i.e. water) reveals amounts of formaldehyde of less than 5 ppm, preferably 0 ppm. Formaldehyde-free microcapsules may be obtained by avoiding formaldehyde or formaldehyde-generating (e.g. generation by decomposition on storage) ingredient in capsule manufacturing, notably ingredients to be used for the manufacture of microcapsule polymeric shell.

The plurality of microcapsules may represent more than 0.01%, such as more than 0.05%, for example more than 0.1% and less than about 5%, for example less than 3%, for example less than 2% by weight over the weight of the composition.

Suitable microcapsules are those whose polymeric shell has solubility in any of water, ethanol or dodecane of less than 1.0g/L at 25°C.

Suitable microcapsules are those whose polymeric shell is obtainable by any one of:
A) condensation reactions such as condensing an isocyanate and a polyamine, or condensing a silyl ether such as tetraethoxysilane and water,
B) free radical polymerization reactions such as polymerizing a monomer blend including vinyl monomers, or
C) coacervation of pre-formed polymers followed by crosslinking of the coacervates by using a crosslinker thereby forming covalent bonds.

As to option A), US8299011 and patents cited therein disclose examples of polyurethane condensation. US6303149 discloses examples of alkoxysilane condensations with water. WO2010102830 discloses phenol aldehyde and amine condensations with aldehydes having more than one carbon atom. As to option B), examples of free radical polymerization can be found in EP2620211 and the background art cited therein. As to option C), examples can be found in US2800457 and US6325951.

All the above methods typically generate a water dispersion of a plurality of microcapsules. This dispersion - which is also known as "microcapsule slurry" - may be further processed in order to incorporate the plurality of microcapsules into the final rinse-off cosmetic composition.

Suitable microcapsules are those whose polymeric shell is obtainable by polymerizing a monomer blend (1) comprising:
i) a monomer (I) selected from the group consisting of:
   - a monoethylenically unsatured monomer,
   - dimethyldiallyl ammonium chloride (DMDAAC), and
   - mixtures thereof
   and
ii) a monomer (II) which is a polyethylenically unsatured monomer selected from the group consisting of divinylbenzene, trivinylbenzene, a C₂-C₂₄ alkyl di- or polyester of (meth)acrylic acid, a C₂-C₂₄ alkyl di- or polyamide of (meth)acrylic acid and mixtures thereof,
wherein monomer (I) is present in an amount comprised between 30% and 80% by weight over the combined weight of monomers (I) and (II) in the blend and monomer (II) is present in an amount comprised between 20% and 70% by weight over the combined weight of monomers (I) and (II) in the blend.

Specific examples of perfume-containing microcapsules obtainable by polymerizing monomer blend (1) can be found in EP2620211, filed by the present applicant.

For example, monomer (I) is selected from the group consisting of: dimethyldiallyl ammonium chloride, acrylic acid, methacrylic acid, maleic acid, itaconic acid, 2-(diethylamino)ethyl methacrylate, dimethylaminoethyl methacrylate, 2-(tert-butylamino)ethyl methacrylate, N-[3-(dimethylamino)propyl]methacrylamide, 3-trimethylammonium propyl methacrylamide chloride, methyl methacrylate, ethyl methacrylate, n-propyl methacrylate, isopropyl methacrylate, tert-butyl methacrylate, isobutyl methacrylate, n-butyl methacrylate, methacrylamide, benzyl methacrylate, isobornyl methacrylate, cyclohexyl methacrylate, tetrahydrofuryl methacrylate, glycidyl methacrylate, 2-hydroxyethyl methacrylate, hydroxypropyl methacrylate, poly(ethylene glycol) methyl ether methacrylate, 2-ethyl(2-oxoimidazolidin-1-yl)methacrylate, acryloxyethyltrimethyl ammonium chloride, methacryloxyethyltrimethyl ammonium chloride, triethylene glycol methyl ether methacrylate, PEG300 methacrylate methyl ether and mixtures thereof. Isomers of the above compounds are also included. For example, the monoethylenically unsatured monomer (I) may be selected from the group consisting of: (meth)acrylic acid, methyl methacrylate, ethyl methacrylate, isopropyl methacrylate, isobornyl methacrylate, 2-hydroxyethyl methacrylate, 2-hydroxypropyl methacrylate, 3-hydroxypropyl methacrylate, glycidyl methacrylate, triethylene glycol methyl ether methacrylate; PEG300 methacrylate methyl ether, and mixtures thereof.

Monomer (II) may comprise, such as consist of, one or more monomers selected from the group consisting of 1,4-butylene glycol dimethacrylate, 1,3-butylene glycol dimethacrylate, pentaerythritol trimethacrylate, glycerol trimethacrylate, 1,2-propylene glycol dimethacrylate, 1,3-propylene glycol dimethacrylate, ethylene glycol dimethacrylate, diethylene glycol dimethacrylate, glycerol dimethacrylate, trimethylolpropane trimethacrylate, ethoxylated pentaerythritol tetramethacrylate, divinylbenzene, and trivinylbenzene. These monomers may be present in an amount of at least 10% by weight over the combined weight of all monomers (II) present in the blend. Monomer (II) may comprise at least, such as consist of, 1,4-butylene glycol dimethacrylate or at least ethylene glycol dimethacrylate.

In another example, monomer (I) may be a combination of:
ia) between 50% and 100% by weight over the weight of the combination of a neutral monomethacrylate monomer (Ia) having a solubility in water at pH 7 and 20ºC equal to, or more than 2g/100ml,
ib) between 0% and 50% by weight over the weight of the combination of another neutral monoethylenically unsatured monomer (Ib), and
ic) between 0% and 15% by weight over the weight of the combination of a ionized or ionizable monoethylenically unsatured monomer (Ic).

The neutral monomethacrylate monomer (Ia) may be selected from the group consisting of 2-hydroxyethyl methacrylate, 2-hydroxypropyl methacrylate, 3-hydroxypropyl methacrylate, glycidyl methacrylate, poly(ethylene glycol) methyl ether methacrylate and mixtures thereof.

Monomer (Ib) may be selected from methyl methacrylate, ethyl methacrylate, n-propyl methacrylate, isopropyl methacrylate, tert-butyl methacrylate, isobutyl methacrylate, n-butyl methacrylate, benzyl methacrylate, isobornyl methacrylate, cyclohexyl methacrylate, tetrahydrofurfuryl methacrylate, mono(ethylene glycol) methyl ether methacrylate, di(ethylene glycol) methyl ether methacrylate, and mixtures thereof. For example, monomer (Ib) may be selected from methyl methacrylate and/or ethyl methacrylate. Preferably, monomer (Ib) includes at least methyl methacrylate. Preferably, monomer (Ib) includes at least ethyl methacrylate.

Examples of monomer (Ic) are (meth)acrylic acid, 3-(methacryloylamino)propyl]trimethylammonium chloride (MAPTAC), dimethyldiallyl ammonium chloride, maleic acid, itaconic acid, 2-(diethylamino)ethyl methacrylate, dimethylaminoethyl methacrylate, 2-(tert-Butylamino)ethyl methacrylate, N-[3-(dimethylamino)propyl]methacrylamide, acryloxyethyltrimethyl ammonium chloride, 2-ethyl(2-oxoimidazolidin-1-yl)methacrylate and mixtures thereof. Preferred examples are methacrylic acid and/or 3-(methacryloylamino)propyl]trimethylammonium chloride. Suitable microcapsules are also those whose polymeric shell is obtainable by polymerizing a monomer blend (2) comprising:
i) between 20% and 75% by weight over the combined weight of compounds (I) to (III) in the blend of a compound (I) which is a monomer (I) as defined above,
ii) between 20% and 70% by weight over the combined weight of compounds (I) to (III) in the blend of a compound (II) which is is a monomer (II) as defined above, and
iii) between 0.01% and 10% by weight over the combined weight of compounds (I) to (III) in the blend of a compound (III) which is a silane compound,
wherein the monomer blend further includes solid colloidal particles having an average primary particle size comprised between 5nm and 1µm.

Compound (III) may comprise, such as consist of, methacryloxypropyltrimethoxysilane and/or methacryloxypropyltriethoxysilane.

A suitable plurality of microcapsules is obtainable by a free radical polymerization process including the following steps:
a) preparing an oil-in-water emulsion having an oil phase and a water phase, said emulsion comprising:
   - a polymerization initiator,
   - a perfume composition,
   - the monomer blend,
   - an emulsifier, and optionally
   - one or more further ingredients to be encapsulated
b) triggering polymerization within the emulsion obtained in step a),
c) letting the polymerization propagate thereby obtaining a plurality of microcapsules.

The plurality of microcapsules is typically obtained in the form of dispersion in water.

A suitable plurality of microcapsules is also obtainable by a free radical polymerization process including the following steps:
a) providing an oil-in-water emulsion having an oil phase and a water phase, said emulsion being obtainable by mixing:
   - an oil soluble polymerization initiator,
   - a perfume composition,
   - a monomer blend as presently defined, and
   - a protective colloid,
b) triggering polymerization within the oil phase of the emulsion obtained in step a),
c) letting the polymerization propagate thereby obtaining a plurality of microcapsules.

The plurality of microcapsules is typically obtained in the form of a dispersion in water.

This water-based dispersion (also referred to as "slurry") functions thus as a concentrated fluid which may be added directly to the composition or to e.g. the aqueous carrier as presently defined in order to obtain the final rinse-off cosmetic composition. Since the addition entails a substantial dilution of the slurry components, microcapsules are typically contained in slurries in amounts that are higher than the target amount in the final product.

The perfume (also herein referred to as "perfume composition") to be encapsulated typically includes a fragrance, i.e. an olfactively active (i.e. odoriferous) material typically but not necessarily providing a pleasant smell. For example, the perfume composition comprises at least two, such as at least four, or at least eight distinct fragrances. Fragrance typically used in the field of perfumery and suitable for the purposes of the present disclosure are described more fully in S. Arctander, Perfume Flavors and Chemicals 1969, Vols. I and II, Montclair, N.J and in Allured's Flavor and Fragrance Materials 2007 ISBN 978-1-93263326-9 published by Allured Publishing Corp. The term fragrance comprises both naturally occurring as well as synthetic fragrances known for use in perfumes.

It may be preferred that the mixture of all fragrances in the perfume composition taken together does not display a solid-liquid phase transition at a temperature comprised between -20°C and 120°C.

It may be preferred that each of the fragrances in the perfume composition independently consists of a single, typically organic, molecule or a mixture of distinct molecules ("perfumery molecules"). Contrary to optical isomers, structural and geometric isomers of a given molecule are considered distinct molecules for the purposes of the present disclosure. Alternatively, each of the fragrances in the perfume composition independently comprises less than 50 distinct perfumery molecules, preferably less than 40 distinct perfumery molecules, more preferably less than 30 distinct perfumery molecules.

For example, each of the perfumery molecules may have a molecular weight greater than 100 amu, preferably greater than 125 amu and lower than 325 amu, preferably lower than 300 amu, more preferably lower than 275 amu.

For example, each of the perfumery molecules may have a boiling point comprised between about 80ºC and 400ºC, such as between about 100ºC and 350ºC when measured at 760 mm Hg.

Advantageously, each of the perfumery molecules is independently selected from the following list of so-called "bulky molecules" and consisting of: methyl n-{4-(4-hydroxy-4-methylpentyl)-3-cyclohexen-1-methylene}-anthranilate, endo 4-(5-methyll-5-norbornen-2-yl)-pyridine, 2,5,5-trimethyl-2-phenyl-1,3-dioxane, isocamphyl cyclohexanol, 2-(2,4-dimethyl-3-cyclohexyl)-5-methyl-5-(1-methylpropyl)-1,3-dioxane, vanillin propylene glycol acetal, indol-hydroxycitronellal schiff s base, dimethyl-2-(5,5,8,8-tetramethyl-5,6,7,8-tetrahydro-naphthalen-2-yl)1,3-dioxolane, 2-cyclohexylidene-2-phenylacetonitrile, benzyl cinnamate, benzyl eugenol, cinnamyl anthranilate, cinnamyl cinnamate, cinnamyl phenyl acetate, 4-methyl-2-phenyltetrahydro-2H-pyran, dibenzyl ketone, 2-methyl-1,5-dioxaspiro[5.5]undecane, 2,2,3,7,7-pentamethylspiro(1,3-dioxan-5,2-norcarane), decahydro-spiro[furan-2(3H),5-(4,7-methano-5H-indene)],3,3-dimethyl-1,5-dioxaspiro[5.5]undecane,4-methyl-1-oxaspiro[5.5]undecene, 8-methyl-1-oxaspiro[4.5]decan-2-one, methyl naphthyl ketone, isobutylquinoline, 6,7-dihydro-1,1,2,3,3-pentamethyl-4(5H)-indanone, tricyclodecenyl acetate and its homologues tricyclodecenyl propionate and tricyclodecenyl isobutyrate, cedryl methyl ether, 4-(1,3-benzodioxol-5-yl)butan-2-one, 3a-ethyl dodecahydro-6,6,9a-trimethylnaphtho[2,1-b]-furan, 2,5,5-trimethyl-octahydronaphthalen-2-ol, α-cedrene, 8-cedren-13-ol, cedryl methyl ether, α-methyl-3,4-methylenedioxy hydrocinnamic aldehyde, ethyl tricyclo[5.2.1.0]decan-2-carboxylate, 1,4-cineole (470-67-7), 1,8-cineole, borneol, bornyl acetate, isoborneol, isobornyl acetate, isobornyl formate, isobornyl methyl ether, isobornyl propionate, 2-ethyl-5(or 6)-methoxybicyclo[2.2.1]heptane and 1-ethyl-3-methoxytricyclo[2.2.1.02,6]heptane, 2-ethylidene-6-isopropoxybicyclo[2.2.1]heptane, 5(or 6)-(methylnorborn-5-en-2-yl)-2-menthyl-1-en-3-ol, 2-[(1,7,7-trimethybicyclo[2.2.1]hept-2-yl)oxy]-ethanol, 7 acetyl-1,2,3,4,5,6,7,8-octahydro-1,1,6,7-tetramethyl-naphthalene, patchouli alcohol, isobornyl cyclohexanol, 2,2,7,7-tetramethyltricyclo[6.2.1.01, 6]undecane-5-one, octahydro-7,7,8,8-tetramethyl-2,3b-methano-3bh-cyclopenta(1,3)cyclopropa(1, 2)benzene-4-methyl acetate, nootkatone, 9-ethylidene-3-oxatricyclo[6.2.1.02, 7]undecane-4-one, cedryl methyl ether, α-pinene, β-pinene, 5,6,7,7a-tetrahydro-4,4,7a-trimethyl-2(4h)-benzofuranone (dihydroactinidolide), 1,3-dimethyl-8-(1-methylethyl)-tricyclo[4.4.0.0.02,7]dec-3-ene (α-copaene), camphene, 4-acetyl-6-tertiary-butyl-1,1-dimethyl indane, 5-acetyl-1,1,2,6-tetramethyl-3-isopropyl-dihydroinden, beta-naphthyl isobutyl ether, decahydro-beta-naphthyl acetate, 6-methoxydicyclopentadiene carboxaldehyde, 4-methyl tricyclo[6.2.1.0]undecan-5-one, 4,4a,5,8,8a-hexahydro-3',7'-dimethylspiro(1,4-methanonaphthalene-2(1H), 2'-oxirane), dodecahydro-3a,6,6,9a-tetramethyl-naphto[2,1-b]furan, 6-ethylidene octahydro-5,8-methano-2H-1-benzopyran-2-one, para-tertiary-butyl-cyclohexanol, ortho-tertiary-butyl-cyclohexanol, para-tertiary-butyl-cyclohexanone, γ-ionone, β-damascenone, 4 acetoxy-4-methyl-2-propyl-tetrahydro-2h-pyran, ortho tertiary amyl cyclohexanyl acetate, 2, 4-dimethylcyclohexanemethanol, ethyl acetoacetate propylene glycol acetal, isocyclogeraniol, 5-methyl-3-butyltetrahydropyran-4-yl acetate, fenchol, (-)-2-ethyl-4-(2,2,3-trimethyl-3-cyclopenten-1-yl)-2-buten-1-ol, ethylacetoacetate ethyleneglycol ketal, nopol, nopyl acetate, 2,6,6-trimethyl-1-cyclohexen-1-acetoaldehyde, 2,4,6-trimethyl-3-cyclohexene-1-carboxaldehyde, 2,4,6-trimethyl-3-cyclohexene-1-methanol, 3-methyl-5-propyl-2-cyclohexen-1-one, dynascone (firmenich), α-iso-methyl-ionone, 3,3-dimethyl-5-(2',2',3'-trimethyl-3'-cyclopenten-1'-yl)-4-penten-2-ol, 2,2-dimethyl-6-methylene cyclohexane carboxylic acid methyl ester, 5-pentyl-2,2,5-trimethylcyclopentanone, 2,2,6-trimethyl-α-propyl-cyclohexanepropanol, 2-tert-butyl cyclohexyl oxy-2-butanol, myrac aldehyde, 2-methyl-4-(2,6,6-trimethyl-1-cyclohexen-1-yl)-2-butenal, 2,2,5-trimethyl-5-pentylcyclopentanone, β-2, 2,3-tetramethyl-3-cyclopentene-1-butanol, 2-methyl-4-(2,2,3-trimethyl-3-cyclopenten-1-yl)-2-buten-1-ol, 3-methyl-5-(2,2,3-trimethyl cyclopent-3-en-1-yl-pentan-2-ol, 4-tert-pentylcyclohexanone, 4-(1-ethoxyvinyl)-3,3,5,5-tetramethylcyclohexanone, ethyl 2-tert-butyl cyclohexyl carbonate, methyl 2-hexyl-3-oxocyclopentanecarboxylate, 3-oxo-2-(2-cis-pentenyl)cyclopentane acetic acid methyl ester, 2-pentyl-3-methyl-2-cyclopenten-1-one, 3-methylcyclopentadecanone, 3-methylcyclopentadecenone, 3-methylcyclopentadecanol, cyclopentadecanone, (Z)-4-cyclopentadecen-1-one, trimethyl-oxabicyclotridecadiene, 15-pentadecenolide, 12-methyl-14-tetradec-9-enolide, oxacycloheptadec-7(or 10)-en-2-one, [3 (or 4)-cycloocten-1-yl]methyl carbonate, methyl 2,6,10-trimethylcyclododeca-2,5,9-trien-1-yl ketone, cyclodecyl methyl ether, ethoxyymethoxycyclidodecane, 2-methyl-3-(4-tert-butylphenyl)propanal, 2,5,5-trimethyl-2-phenyl-1,3-dioxane, 4-tert-butylbenzennpropionaldehyde, dimethybenzylcarbinyl acetate, 5-phenyl-5-methyl-3-hexanone, 1,3,5-trimethoxybenzene, acetyl eugenol, acetyl vanillin, 3,4-dimethoxybenzoic acid, and 3,4-methylenedioxybenzyl acetate veratraldehyde.

The use of "bulky" molecules as presently discussed can be associated to a reduced leakage of the perfume composition from the microcapsules into the composition, thereby further improving the effective deposition of the same. More information regarding bulky molecules, examples of fragrances and microcapsules containing the same and an in-depth disclosure of the advantages of using the same can be found for example in EP1894603, filed by the present applicant.

More information and examples of other perfume compositions particularly advantageous in that they support good encapsulation efficiency and reduced perfume leakage from conventional microcapsules such as aminoplast microcapsules can be found in EP1767185, filed by the present applicant.

Irrespective of the manufacturing method chosen to manufacture the plurality of microcapsules, the perfume composition is generally subjected to an emulsification followed by encapsulation (according to various techniques). It is commonly accepted that quantitative definitions within the perfume composition are assessed on the fragrance(s) to be encapsulated prior to emulsification and encapsulation. This approach makes it possible to have a common reference to reliably compare perfume compositions, while providing practical advantages as it entails assessing the quantitative definitions on larger volumes thereby making the entire assessment easier and potentially more accurate. For example, difficulties (hence, possible errors) associated with extracting very small amounts of perfumes from e.g. already formed microcapsule dispersions or final products (precisely as rinse-off cosmetic compositions) are avoided. Possible modifications in perfume quantitative composition due e.g. to fragrance emulsifiability and encapsulation efficiency are also designed around. Suitable perfume compositions to be used in the context of the present composition can thus be obtainable by emulsifying and encapsulating a fragrance, such as a mixture of distinct fragrances, that are quantitatively as defined below.

Advantageously, prior to emulsification and encapsulation, between 60% and 100% by weight of all fragrances taken together consists of five or more distinct perfumery molecules each independently selected within the list of "bulky molecules" as defined above.

It may also be advantageous that, prior to emulsification and encapsulation, the fragrance represents at least 40%, such as at least 60%, for example at least 80%, such as at least 90% by weight over the weight of the perfume composition. The balance of the perfume composition may be represented by perfumery acceptable solvents and/or benefit agents. It may also be advantageous that, prior to emulsification and encapsulation all fragrances taken together have a ClogP greater than 1.0 (limit not included), more preferably equal to, or greater than 2 but equal to, or lower than for example 7, or equal to, or lower than for example 6, or equal to, or lower than for example 5, or equal to, or lower than for example 4.5. For example, preferred fragrances have a ClogP comprised between 2 and 5, for example in the range comprised between 2 and 4.5. This may be done by mixing distinct fragrances, each meeting the ClogP values disclosed above.

In case multiple fragrances are admixed, it is advantageous that each of the fragrances present be as defined above.

Suitable perfume compositions may further include a perfumery acceptable solvent and/or a benefit agent.

Examples of suitable solvents are a water-immiscible solvent, for example a solvent having water solubility of less than 10g/L, for example one or more of water insoluble hydrocarbon solvents (such as the Isopar® family from ExxonMobil), benzyl benzoate, isopropyl myristate, dialkyl adipates, citrate esters (such as acetyl triethyl citrate and acetyl tributyl citrate) and diethyl phthalate.

Advantageously, the perfume composition does not contain water miscible solvents (e.g. solvents with water solubility of more than 10g/100ml), such as propylene glycol dipropylene glycol, and butylene glycols.

The benefit agents may include:
- agents which suppress or reduce malodour and its perception by adsorbing odour such as zinc ricinoleate,
- agents improving microcapsule physical-chemical properties such as sucrose octa-acetate or sucrose hexabutyrate di-acetate,
- gelling agents such as hydroxy fatty acids or the Sylvaclear™ range of materials available from Arizona Chemicals,
- agents which provide a warming or cooling effect such as cyclohexane carboxamide N-ethyl-5-methyl-2-(1-methylethyl); N-2,3-trimethyl-2-isopropylbutamide; menthyl lactate; (-)-menthoxypropane 1,2-diol,
- insect repellents such as ethylbutylacetylaminopropionate; N,N-diethyl toluamide; 1-piperidinecarboxylic acid; 2-(2-hydroxyethyl)-1-methylpropyl ester; p-menthane-3,8-diol,
- antimicrobial agents such as triclosan™ compound having CAS N° 3380-34-5, or the methyl, ethyl, propyl and butyl para hydroxy benzoate esters, and/or
- UV absorbers such as octyl methoxycinnamate, butylmethoxydibenzoylmethane, and bis ethylhexyloxyphenolmethoxyphenyltriazine.

As already disclosed above, it is preferable that the microcapsules have a density comprised between 0.85 g/ml and 1.1 g/ml when measured according to the capsule Density Method disclosed in the "Methods" section, below.

To combine proper emulsifiability with appropriate microcapsule densities, it may be advantageous to have a perfume composition which is obtainable by emulsifying and encapsulating a fragrance including, on a weight/weight basis:
- between about 20% and 100%, preferably 40-100% and more preferably 60-100% by weight over the weight of the fragrance of a cyclic perfumery molecule having a density comprised between 0.950g/cm³ and 1.500g/cm³ at 20ºC and a ClogP value comprised between 1.00 and 6.00;
- between about 0 and 80% of a perfumery molecule having density comprised between 0.63g/cm³ and 0.950g/cm³ (upper limit excluded) measured at 20ºC and a ClogP value comprised between 1.00 to 6.00.

In case a solvent and/or a benefit agent are present, it may be advantageous that, prior to emulsification and encapsulation, the perfume composition comprises a fragrance as defined above and between about 0 and 50% of a solvent and/or benefit agent, each having a density comprised between 0.95g/cm³ and 1.50g/cm³ at 20ºC.

When used in the present disclosure, ClogP refers to the octanol/water partitioning coefficient (P) of a given ingredient, such as perfumery molecules. The octanol/water partitioning coefficient of a perfumery molecule is the ratio between its equilibrium concentrations in octanol and in water. The partitioning coefficients of fragrances are more conveniently given in the form of their logarithm to the base 10, logP. The logP values of many perfumery molecules and complex fragrances have been reported; for example, the Pomona92 database, available from Daylight Chemical Information Systems, Inc. (Daylight CIS), Irvine, Calif., contains many, along with citations to the original literature. The "calculated logP" (ClogP) is determined by the fragment approach of Hansch and Leo (cf., A. Leo, in Comprehensive Medicinal Chemistry, Vol. 4, C. Hansch, P. G. Sammens, J. B. Taylor and C. A. Ramsden, Eds., p. 295, Pergamon Press, 1990, incorporated herein by reference). The fragment approach is based on the chemical structure of each perfume composition, and takes into account the numbers and types of atoms, the atom connectivity, and chemical bonding. The ClogP values, which are the most reliable and widely used estimates for this physicochemical property, are preferably used instead of the experimental logP values in the selection of fragrances which are useful in the present disclosure. There are several alternative methods of calculating or estimating logP values which can show some variation in values. Even calculations within a given set of software may change over time as the algorithms are modified to give results which are closer to measured values. To remove any uncertainty the ClogP values reported herein are most conveniently calculated by the Daylight program version 4.94 available from Daylight Chemical Information Systems. The ClogP values are preferably used instead of the experimental logP values in the selection of fragrances which are useful in the present disclosure. Guidance for determining the composition of natural oils or extracts can be found in the ESO 2000 database published by BACIS (Boelens Aroma Chemical Information Service, Groen van Prinsterlaan 21, 1272 GB Huizen, The Netherlands).

The rinse-off cosmetic compositions presently disclosed may be prepared by adapting the "Shampoo Preparation Method" disclosed in the "Methods" section below. This method can be adjusted in order to e.g.
- replace specific ingredients mentioned therein with other equivalent/alternative solutions as presently disclosed,
- vary process parameters such as temperatures, mixing conditions, and order of ingredient addition as is well known to those experienced in such processes,
- remove optional ingredients mentioned therein but that might not be desired in a given composition, and/or
- be applicable to other rinse-off cosmetic products such as body washes and shower gels.

In a further aspect, the present disclosure discloses the use of an ingredient selected from the group consisting of a hydrophobically modified copolymer, a hydrophobically modified crosspolymer, a non-hydrophobically modified crosspolymer, and mixtures thereof, as suspending agent in a rinse-off cosmetic composition, said composition comprising an aqueous carrier and a plurality of perfume-containing, formaldehyde-free microcapsules dispersed therein, wherein the microcapsules have an average particle size as median volume particle size D(v;0.5) value comprised between about 5 and 30 microns, for example between about 5 and 25, such as between about 5 and 20 microns or between 7.5 and 15 microns or between 10 and 15 microns. The liquid rinse-off cosmetic composition may be as disclosed in the present disclosure. The plurality of formaldehyde-free perfume-containing microcapsules may be as disclosed in the present disclosure. Further embodiments and advantages of the present invention will become apparent to a skilled reader in light of the examples provided below.

### Methods

Below, and unless otherwise indicated, % are to be understood as weight %.

### Shampoo test protocol on hair swatches

Hair swatches are mid brown European natural hair (ex Hugo Royer Int. Ltd.). Prior to testing the hair is prepared by immersing in a bath of ethanol for 30min to remove any free and encapsulated fragrance. Then the swatches are rinsed thoroughly with water. The hair swatches are then washed with an unfragranced shampoo of example 3 as follows: i) apply about 2ml of shampoo onto the hair swatch and massage it in for 30s; ii) rinse the hair swatch for 30s with a stationary shower rinse (37°C, 200 ml/s); iii) then squeegee 4 times between 2 fingers along the length of the hair swatch to remove excess of water; iv) comb to detangle and dry the hair swatch at ambient conditions by hanging on a rack. Comb a hair swatch of about 10g. Dip it in water (300ml, 37°C) three times for 5 seconds each time. Then remove excess water by squeegeeing the swatch through the fingers twice. Check the weight which should be about 1.5 times the original swatch weight +/-10%. Apply 0.5 ml of the test product onto the wet hair swatch from a syringe. Spread the product along the swatch and massage the hair swatch for 5s by gentle circular motions between gloved hands to generate some foam. Holding the swatch in 2 fingers by the metal rinse the hair swatch for 10s with a stationary shower rinse (37°C, 200 ml/s) turning the hair swatch once. There is no manipulation of the hair swatch (thus 5s rinsing for each side). Press the hair swatch in one hand to remove excess water then squeegee the swatch through two fingers to remove more water. Then wrap the swatch in absorbent paper and dab gently to remove more water. Typically the swatch should now weigh 1.3 times +/- 10% more than the original dry weight. Comb the hair swatch 3 times to remove tangles and hang on a rack to dry at ambient temperature and humidity. The time of starting to dry is time zero for assessment of the fragrance strength.

### Fragrance intensity assessment (products intended for application onto hair)

The standard assessment protocol is for expert evaluators to assess the fragrance intensity of each of 3 hair swatches before combing and then after combing each swatch 3 times. Usually this is done at time zero plus 4 hours (t+4h). The same hair swatches may be assessed again at time zero plus 6 hours (t+6h) and at time zero plus 24 hours (t+24h). The protocol allows for the residual fragrance from any perfume directly incorporated in the product to be assessed separately before fragrance is released from the capsules. In the following examples there is no free fragrance so only the average fragrance intensity scores after combing are given. Also, as might be expected, the strength of fragrance reduces with successive combing so the intensities reported at time zero +4 hours will be stronger than time zero plus 6 hours which in turn will be stronger than time zero plus 24 hours. Thus to simplify the results only the time zero +4 hour results are reported. Fragrance strength is measured using an interval scale from 0 to 5 in which the scale points have the following meanings.
0 no fragrance detected
1 poor fragrance strength
2 fair fragrance strength
3 good fragrance strength
4 very good fragrance strength
5 excellent fragrance strength.

### Shower gel test protocol on forearms

The design of shower gel tests is to compare both a test and a control product on a single person with the test product on one forearm and the control product on the other. At least 3 people act as test subjects per experiment and evaluators assess the fragrance intensity as soon after rubbing as possible.

Before any test products are applied the whole forearm arm is washed from wrist to elbow with an unfragranced shower gel of example 4a and dried with a paper towel. Wet all the back of the forearm for 5 sec with running tap water (37°C, 200 ml/s) - back and forth with the tap between the wrist and the elbow. Apply 0,5ml of test product by syringe on the skin (at mid-forearm). Wash with a gloved hand for 15 sec using a circular motion over an distance of approximately 15cm across the forearm. Rinse for 5 sec with running tap water (37°C, 200 ml/s) - back and forth with the tap between the wrist and the elbow. Remove excess water with a paper towel.

### Fragrance intensity assessment (products not intended for application onto hair)

Assessment is made 2 hours after product application with assessments before and after rubbing to determine whether there is any residual free fragrance which may have been present in a product or fragrance leaking from capsules. Evaluators judge the perfume strength using the same 0-5 point scale as used for hair swatches. After assessing any fragrance on the forearm the area of skin to which the test product was applied is rubbed with a gloved hand and the fragrance strength assessed again. Further assessments may be made at t+4h and t+6h.

### Product storage stability

Samples of products are formulated and stored in sealed clear glass bottles and stored at 40°C. After 2 weeks, 4 weeks, 6 weeks, 8 weeks, 10 weeks and 12 weeks the samples are removed inspected visually and the stability of the microcapsule suspension (herein also referred to as capsule stability) is assessed against the 4 point scale of Table 2 (half points are possible if a given sample is between two criteria):

**Table 2**

| **Score** | **Criterion** |
|---|---|
| 0 | Clear layer in sample |
| 1 | Some suspended capsules throughout sample |
| 2 | Acceptable suspension: limited capsule sedimentation or floating |
| 3 | Total suspension: capsules do not sediment or float, homogeneous sample |

### Viscometry

Products having viscosities in the normal product range are measured on a Brookfield model LVDV-I+ at 20 rpm with spindle 25 and thermostatted at 20°C. All viscosity measurements disclosed in the present disclosure were made with kit SSA (small sample adapter) with the Water Jacket Assembly SC4-45Y. For products with higher or lower viscosities which require different spindles or speeds the operating manual is followed and the viscometer is calibrated with Fluid 100 at 25°C at fortnightly intervals.

### Capsule density method

Capsule slurry density is measured using a Gay Lussac density bottle to weigh a known volume of capsule slurry. Then the percentage of capsules in the slurry (dry weight %) is measured by drying an accurately weighed aliquot of approximately 2g of capsule slurry into a beaker and heating the beaker in an oven at 120°C for 1 hour. The beaker is allowed to cool and weighed. The residual weight divided by the initial slurry weight gives the percentage of capsules within the slurry. Since the free liquor density is known to be very close to 1.000kg/m³ the capsule density can be therefore calculated.

### Examples

### Example 1 - Fragrance composition

**Table 3**

| **Ingredient** | **CAS No** | **Amount (wt%)** |
|---|---|---|
| Verdox | 88-41-5 | 55.0 |
| Diphenyl oxide | 101-84-8 | 10.0 |
| Aldehyde C-11 Undec | 112-45-8 | 10.0 |
| Dihydromyrcenol | 18479-58-8 | 10.0 |
| Styrallyl acetate | 93-92-5 | 10.0 |
| Eucalyptol | 470-82-6 | 5.0 |

The above fragrance was subjected to encapsulation according to the following "capsule preparation method".

### Example 2 - Capsule Preparation Method

A 10% poly(vinyl alcohol) aqueous solution was prepared in advance by dissolving poly(vinyl alcohol), hydrolyzed to 87-89%, M_{w}=85000-124000 g/mol in water (available from Sigma Aldrich). An oil phase is prepared by mixing 150 g of the fragrance, 21.8 g of methacrylic acid, 8.7 g of methyl methacrylate, 24.0 g of 1,4-butane diol dimethacrylate, 1.5 g of benzoyl peroxide, 75% in water. The mixture is stirred in order to obtain a monophasic, homogeneous and transparent phase. A dispersion of silica in water is prepared separately by stirring during 5 min 1.25 g of Aerosil® R816 silica, 200 g of water containing 100 mg/L of sodium bicarbonate. The oil phase and the dispersion of silica in water are stirred together at 7000 rpm for 2 min using a high-shear mixer (e.g. Ystral X 10/20 E3-1050 W equipped with a Dispermix head of diameter 40/54 mm). The mean particle size of the resultant emulsion is determined according to the method disclosed above. 360 g of the emulsion are placed into a sealed 500 mL-batch reactor equipped with a condenser, a thermometer, a bottom outlet valve and an anchor stirrer. 43 g of 10% poly(vinyl alcohol) aqueous solution is added and the mixture is stirred during 10 min. Throughout the process, the mixture is stirred at 250 rpm. The temperature is first fixed at a temperature of 25 °C for 30 min and the temperature is then increased to 80 °C within one hour. The mixture is kept at 80°C and samples of 2 g are withdrawn by using the bottom outlet valve every 30 min in order to determine the monomer conversion. The mixture is kept during a sufficient time at 80°C to obtain a monomer conversion at least equal to 85% or higher than 85%. The resultant microcapsule dispersion is cooled to room temperature within 1 hour. Mean particle sizes of the resultant microcapsule dispersion are determined according to the capsule particle size measurement method.

### Example 3 - Shampoo Formulation

A plurality of microcapsules obtained as per the Capsule preparation method was then used in a shampoo comprising the following ingredients (Table 4). Hereinafter, the language "deposition polymer" may be interchangeably used for "polymer deposition aid".

**Table 4**

| **Ingredient (Trade Name)** | **INCI Name** | **Active in formulation (wt%)¹** |
|---|---|---|
| Suspending agent | As specified | As specified |
| Texapon N70 | Sodium Laureth Sulfate | 13.0 |
| Dehyton P45 | Cocoamidopropyl Betaine | 2.0 |
| Fragranced capsule | As specified | As specified |
| Polyox WSR N-60 | PEG 45M | 0.05 |
| Pricerine | Glycerin | 1.0 |
| Deposition polymer | As specified | As specified |
| Sodium hydroxide | Sodium Hydroxide | 0.1 |
| Sodium benzoate | Sodium Benzoate | 0.5 |
| Citric acid | Citric Acid | as required to reach pH of 5 |
| Sodium Chloride | Sodium Chloride | As required for viscosity of 7,000cps |
| Demineralized water | Aqua | To 100% |

| | | |
|---|---|---|
| 1: Texapon N70 contains only 70% of sodium laureth sulfate surfactant thus to achieve 13.00% of ether sulfate in the formulation i.e. active ingredient it is necessary to add 18.57% of Texapon N70 as supplied to the formulation. | | |

Texapon N70 and Dehyton PK45 are registered trademarks of BASF SE. Polyox WSR n-60 is a registered trademark of The Dow Chemical Company. Pricerine is a registered trademark of Croda International PLC.

### Shampoo preparation method (applicable to any other product like body washes and shower gels):

The shampoo samples in the present disclosure were prepared according to the following general procedure (adapted as necessary).
1. Pre-weigh beaker (so that batch weight can be adjusted at the end) and place on stirrer/hotplate.
2. Add most of the batch demineralized water, leaving out sufficient for making slurries/ washing ingredients into the batch (this will depend on the amount of pre-dispersions, final pH and viscosity adjustments and batch size).
3. Add a few drops of 30% citric acid (recording amount) and start mixing using a Heidolph type overhead stirrer with suitable blade.
4. Add the suspending agent gradually into the vortex and stir until completely dispersed.
5. Add the Texapon N70 or other principle surfactant while stirring continuously but carefully to keep any aeration to a minimum. Continue stirring until homogeneous.
6. Add the Dehyton P45 or any secondary surfactant solution and leave to mix until fully dispersed.
7. Optionally prepare a premix of deposition aid (e.g. Jaguar C17) in water adjusted to pH>10 with NaOH and add to batch, rinsing out the container with a little water.
8. Mix well for a few minutes then adjusts pH using 30% sodium hydroxide or 30% citric acid to the higher end of pH spec.
9. Premix Polyox polymer with the glycerol then add slowly using a disposable pipette into the vortex.
10. Add the preservatives
11. Optionally add any silicones, pearliser etc. and mix until homogeneously dispersed.
12. Adjust to target pH (e.g. pH 5.0) by careful addition of 30% sodium hydroxide or 30% citric acid, noting amounts added.
13. Measure the viscosity of the shampoo (Brookfield) and add sodium chloride solution to adjust to the target value, again noting the total amount added.
14. Re-weigh the beaker + batch and add any further water required to make the batch up to a weight of 98% of formulation leaving a 2% hole for the subsequent addition of fragranced capsules, free fragrance and water to 100% of the final formulation.
15. 24 hours after preparation transfer a small sample into a suitable vessel to check the viscosity. If it is out of the desired spec range, transfer the sample back into the batch and add a little more salt (to thicken) as required.
16. Take a portion of 49g of the product and add the requisite amount of fragranced capsule slurry and add demineralized water to a final weight of 50g for further experiments.

### Example 4 - Exemplary shower gel formulations

The formulations below (Table 5) are a shower gel formulation containing a deposition aid (4b) and the other minus the deposition aid (4a). In neither case was it necessary to add salt to achieve the target viscosity of 7,000cps.

**Table 5**

| **Ingredient** | **Ex 4a (wt%)** | **Ex 4b (wt%)** |
|---|---|---|
| Texapon N70 | 10 | 10 |
| Dehyton PK45 | 4 | 4 |
| Ultrez 20 | 0.6 | 0.6 |
| Fragranced capsule | 1.3 | 1.3 |
| Pricerine | 5.0 | 5.0 |
| Jaguar C13S | 0.0 | 0.2 |
| Sodium hydroxide | 0.18 | 0.18 |
| Sodium benzoate | 0.5 | 0.5 |
| Citric acid | as required to reach pH of 5 | as required to reach pH of 5 |
| Sodium Chloride | 0 | 0 |
| Demineralized water | To 100% | To 100% |

The preparation of the shower gel compositions was the same as the shampoo preparation method given for example 3.

### Examples 5 and 6 - Effect of capsule particle size on perfume performance from a shampoo without (example 5) or with (example 6) a polymer deposition aid

Hair swatches were washed and tested using a shampoo formulation of example 3 containing 0.6% Ultrez 20 as a suspending agent and 0.4% sodium chloride added to achieve the target 7,000cps viscosity. The shampoo did not contain any deposition aids as defined in this document. Capsules prepared according to the method of example 2 with a fragrance of example 1 were prepared at different average particle sizes as indicated in table 6 below. These capsules were dosed in amounts to equal 0.325% of fragrance in each prototype shampoo.

**Table 6**

| **Capsule** | **Size (micron)** | **Dosage (%)** | **Test T+4h** |
|---|---|---|---|
| Cap 1 | 42.0 | 0.99 | 0.8 |
| Cap 2 | 23.8 | 0.99 | 1.7 |
| Cap 3 | 10.7 | 1.3 | 1.8 |

Hair swatches were washed and tested using a shampoo formulation of example 3 containing 0.6% Ultrez 20 as a suspending agent and 0.4% sodium chloride added to achieve the target 7,000cps viscosity. The shampoo also contained 0.2% of Jaguar C17 (a trimethyl ammonium guar ex Solvay) as a deposition aid. Capsules prepared according to the method of example 2 with a fragrance of example 1 were prepared at different average particle sizes as indicated in table 7 below. These capsules were dosed in amounts to equal 0.325% of fragrance in each prototype shampoo.

**Table 7**

| **Capsule** | **Size (micron)** | **Dosage (%)** | **Test T+4h** |
|---|---|---|---|
| Cap 1 | 42.0 | 1.0 | 0.2 |
| Cap 2 | 23.8 | 1.0 | 3.5 |
| Cap 3 | 10.7 | 1.30 | 3.8 |

The polymer deposition aid was found to bring an advantage in terms of perfume performances for small microcapsules whereas no appreciable effect was achieved for bigger microcapsules.

### Example 7 - A comparison of shower gel formulations showing the benefit of a deposition aid

Forearms were washed and tested (Table 8) using shower gel formulations of examples 4a (comparative) and 4b. Shower gel 4b contains 0.2% of Jaguar C13S (a trimethyl ammonium guar) as a deposition aid. Capsules of 10 micron size were prepared according to the method of example 2 with a fragrance of example 1 and dosed in amounts to equal 0.325% of fragrance in each prototype shower gel.

**Table 8**

| **Composition** | **Test T+4h** |
|---|---|
| Cap 3 with Jaguar C13 S - Ex 4b | 3.5 |
| Cap 3 without Jaguar C13 S - Ex4a | 2.5 |

### Example 8 - Effect of different cationic polymers as deposition aids on capsule performance on hair

In the experiment different cationic polymers were dosed at 0.2% by weight into samples of the reference shampoo formulation of example 3. For each prototype product viscosity was adjusted to approximately 7,000 cps by adding 0.6% Carbopol Ultrez 20 and sodium chloride. Capsules of 10 micron average size and with the properties described previously for Cap 3 were dosed into each prototype at 1.3% containing 0.325% of perfume.

**Table 9**

| **Cationic polymer** | **Test T+4h** |
|---|---|
| No polymer | 1.2 |
| Dehyquart CC7 BZ | 1.8 |
| Ucare JR400 | 1.5 |
| No polymer (repeat) | 1.2 |
| Jaguar C17 | 3.2 |

Separate shampoo hair wash tests with prototypes containing other cationic polymers dosed at 0.2% w/w gave the results shown below in Tables 10-11.

**Table 10**

| **Cationic polymer** | **Test T+4h** |
|---|---|
| No polymer | 0.5 |
| SoftCat polymer SX-1300H | 3.7 |
| Ucare JR400 | 1.3 |
| Jaguar C162 | 1 |
| Jaguar Excel | 2.5 |
| Jaguar C17 | 4 |

**Table 11**

| **Cationic polymer** | **Test T+4h** |
|---|---|
| SoftCat polymer SX-1300H | 2.8 |
| Jaguar C500 | 0.8 |
| Jaguar Excel | 2.0 |
| Jaguar C17 | 3.8 |

The example supports the benefit of a cationic derivative of natural polysaccharide polymers, such as guar gum or cellulose based polymers, as deposition aid in capsule performance and, in particular, the general superiority of high molecular weight, high charge density cationic polymers (such as Jaguar C17 and Softcat SX 1300-X).

### Example 9a - Suspending agents and perfume performance in a shampoo formulation

In this experiment capsule Cap 3 was dosed at 1.3% in 3 prototype shampoo formulations of example 3 with varying amounts of the suspending agent (Carbopol Aqua SF1), in the presence/absence of sodium chloride and with 0.2% of Jaguar C17 as a deposition aid. Hair swatches were washed and the fragrance intensity assessed after combing as described in the test method. Results are as per Table 12:

**Table 12**

| **Suspending agent** | **Test T+4h** |
|---|---|
| 0.4% salt +0.6% Ultrez 20 | 3.5 |
| 0% salt + 0.6% Aqua SF1 | 2 |
| 0% salt + 0.9% Aqua SF1 | 1.7 |
| 0% salt + 1.2% Aqua SF1 | 0 |

Thus increasing the proportion of Carbopol Aqua SF1 (INCI Acrylates Copolymer) progressively reduces perfume performance from the capsules. However the dosage of some other types of suspending agents did not adversely affect performance. Table 13 below shows perfume performance at two dosage levels of polymer Ultrez® 10, all other parameters being as above. In this experiment dosage level did not affect performance.

**Table 13**

| **Suspending agent** | **Test T+4h** |
|---|---|
| 0.6% Ultrez 10 no salt | 2.5 |
| 1.0% Ultrez 10 no salt | 2.7 |
| 0.6% Ultrez 20 +0.4% salt | 4.0 |

A similar experiment was performed with another suspending agent Aculyn 22. In this experiment (Table 14) salt was added to the product to maintain viscosity.

**Table 14**

| **Suspending agent** | **Test T+4h** |
|---|---|
| 0.6% Aculyn 22 +0.4% salt | 3.0 |
| 1.2% Aculyn 22 +0.4% salt | 3.7 |
| 0.6% Ultrez 20 +0.4% salt | 4.0 |

At the levels dosed, Aculyn 22 did not show a negative effect on perfume performance.

### Example 9b - Suspending agents and stability in a shampoo formulation

Table 15 below shows the storage stability of several capsules containing different fragrances (thus having different densities) with 0.6% w/w Aculyn® 22 in a shampoo formulation similar to the one of example 3 containing 0.2% Jaguar C17 and 0.4% salt (the only difference with respect to the formulation of example 3 is that the viscosity was 429 cps and was not adjusted to 7000 cps).

**Table 15**

| Aculyn 22 | 2 weeks | 4 Weeks |
|---|---|---|
| TC 105 | 0 | 0 |
| TC088 | 1 | 0 |
| WL631 | 1 | 0 |
| WL604 | 1 | 0 |

The capsule stability results for Aculyn® 22 were not optimal as after storage at 40°C for 2 weeks the capsules mainly separated from the liquid.

Capsule stability was then assessed, still using the method presently disclosed, in a shampoo formulation. The results (Table 16) are stated for samples stored for 12 weeks at 40ºC. In most cases the viscosity of the formulations was adjusted as specified by adding more or less sodium chloride.

**Table 16**

| **Example** | **Suspending polymer** | **Deposition aid** | **Viscosity (cps)** | **Stability after 12w** |
|---|---|---|---|---|
| 9b(A) | Ultrez® 20 0.6% | / | 7368 | 2 |
| 9b(B) | ETD®2050 0.6% | Jaguar C17 0.2% | 755 | - |
| 9b(C) * | Aqua® SF-1 0.6% | Jaguar C17 0.2% | 6216 | 0 |
| 9b(D) | Ultrez® 20 0.6% | Jaguar C17 0.2% | 7680 | 3 |
| 9b(E) | Ultrez® 20 0.6% | Jaguar C17 0.2% | >25000 | 3 |
| 9b(F) | Ultrez® 20 0.6% | Jaguar C17 0.2% | 8496 | 3 |
| 9b(G) | Ultrez® 20 0.6% | PQ7 at 0.2% | 5760 | 2 |
| 9b(H) * | - | Jaguar C17 0.2% | 7361 | 2 |
| 9b(J) | Carbopol® silk 100 0.8% | Jaguar C17 0.2% | 9024 | 3 |
| 9b(K) | Ultrez® 20 0.6% | PQ10 at 0.2% | 7656 | 2 |
| 9b(L) | SepiMax® Zen 0.6% | Jaguar C17 0.2% | 8496 | 3 |

| | | | | |
|---|---|---|---|---|
| * = comparative | | | | |

Ultrez® 20 and SepiMax® Zen are examples of hydrophobically modified crosspolymers as presently defined; Carbopol® silk 100 and ETD®2050 are examples of non-hydrophobically modified crosspolymers as presently defined; Aqua® SF-1 is an example of non-hydrophobically modified copolymers as presently defined.

Jaguar® C17 and PQ10 (i.e. Polyquaternium-10) are examples of a cationic derivative of natural polysaccharide polymers as presently defined; PQ7 means Polyquaternium-7, i.e. an example of a synthetic polymer of a cationic vinyl monomer as presently defined.

Except for examples 9(b)B, (E), (F) and (J), the shampoo composition is as disclosed in example 3 except that the % of sodium chloride was not carefully adjusted but was rather roughly dosed so as to give viscosities approximating (either in defect or excess) the target value of 7,000cps.

In examples 9(b)B and 9(b)J, the same amount of NaCl as in example 9(b)D was used (i.e. 0.4% w/w NaCl) irrespective of the resulting viscosity.

In example 9(b)E the amount of surfactant was increased till 25% w/w (21.7% SLES and 3.3% CAPB, ratio SLES:CAPB is close to ratio primary:secondary surfactant in example 3, i.e. 13% SLES and 2% CAPB) and no NaCl was added. In example 9(b)F, the amount of surfactant was decreased to 9% w/w (7% SLES and 2% CAPB, ratio SLES:CAPB is lower than in example 3) with 0.7% NaCl.

In example 9b(B) instability (with and without capsules) was noted from week 8 on so no assessment was made.

The above results demonstrate that hydrophobically modified crosspolymers generally provide for excellent stability of capsule suspension, in addition to achieving the primary goal of maintaining perfume performances. The results also show that a preference can be generally found for combinations of cationic derivatives of natural polysaccharide polymers, notably derivatives of guar gum, as deposition aids with hydrophobically modified crosspolymers as suspending agents.

### Example 10 - Alternative liquid rinse off compositions

Those listed in the following table 17 are alternative combinations of surfactants and variation in surfactant ratios according to the present disclosure and that can reasonably be used in liquid rinse off compositions as presently disclosed.

**Table 17**

| **Ingredient** | **INCI name** | **Ex10a** | **Ex 10b** | **Ex 10c** | **Ex 10d** |
|---|---|---|---|---|---|
| | | (wt%) | (wt%) | (wt%) | (wt%) |
| Texapon N70 | Sodium Laureth Sulfate | 10.0 | 7.0 | 13.0 | 13.0 |
| Dehyton P45 | Cocoamidopropyl betaine | 2.0 | 2.0 | - | 2.0 |
| Miranol C2M | Sodium cocoamphodiacetate | - | - | 2.0 | - |
| Ultrez 20 | Acrylates/C10-30Alkyl Acrylate Crosspolymer | 0.6 | 0.6 | 0.6 | 0.6 |
| Fragranced capsule Cap 3 | | 1.3 | 1.3 | 1.3 | 1.3 |
| Polyox WSR N-60 | PEG 45M | 0.05 | 0.05 | 0.05 | 0.05 |
| Pricerine | Glycerol | 1.0 | 1.0 | 1.0 | 1.0 |
| Jaguar C17 | Guar Hydroxypropyltrimonium Chloride | 0.2 | 0.2 | 0.2 | 0.2 |
| Sodium hydroxide | | 0.26 | 0.25 | 0.25 | 0.28 |
| Sodium benzoate | | 0.5 | 0.5 | 0.5 | 0.5 |
| Citric acid | | to pH 5 | to pH 5 | to pH 5 | to pH 5 |
| Sodium Chloride | | 0.6 | 0.7 | 0.8 | 0.4 |
| Demineralized water | | To 100% | To 100% | To 100% | To 100% |

Miranol C2m is a trade mark of Solvay S.A.

The fragrance performance of capsule Cap 3 on hair swatches, washed according to the stated method, with the shampoo compositions of examples 10a to 10d is shown in the following table 18.

**Table 18**

| **Product** | **Test T+4h** |
|---|---|
| Ex 10a | 4 |
| Ex 10b | 4.3 |
| Ex 10c | 4.8 |
| Ex 10d | 3.2 |

Thus the capsule size range selected performs across a range of shampoo formulations.

### Example 11

In this example of shampoo composition (Table 19) sodium lauryl ether sulfate (Texapon N70) was replaced with ammonium lauryl ether sulfate (Empicol EAC 70) so as to show that the perfume performance of small capsules on hair swatches remains good.

**Table 19**

| **Ingredient** | **Ex11 (Wt%)** | **Ref (Ex 10d) (Wt%)** |
|---|---|---|
| Empicol EAC 70 | 13.0 | / |
| Texapon N70 | / | 13.0 |
| Dehyton P45 | 2.0 | 2.0 |
| Ultrez 20 | 0.6 | 0.6 |
| Fragranced capsule Cap 3 | 1.3 | 1.3 |
| Polyox WSR N-60 | 0.05 | 0.05 |
| Pricerine | 1.0 | 1.0 |
| Jaguar C17 | 0.2 | 0.2 |
| Sodium hydroxide | 0.40 | 0.28 |
| Sodium benzoate | 0.5 | 0.5 |
| Citric acid | to pH 5 | to pH 5 |
| Sodium Chloride | 2.2 | 0.4 |
| Demineralized water | To 100% | To 100% |

Empicol EAC 70 is a registered trade mark of Huntsman Inc.

Testing the perfume performance of the shampoo composition of example 11 against the reference gave the following fragrance intensity scores (Table 20):

**Table 20**

| **Composition** | **Test T+4h** |
|---|---|
| Example 11 | 5 |
| Ref (Ex 10d) | 5 |

Thus again it has been demonstrated that capsules within the range of the invention have good performances across a range of liquid rinse off formulations.

## Claims

1. A rinse-off cosmetic composition comprising an aqueous carrier and a plurality of perfume-containing, formaldehyde-free microcapsules dispersed in the composition, wherein the composition is liquid, the composition comprises a surfactant and a suspending agent selected from the group consisting of Acrylates/C10-30Alkyl Acrylate Crosspolymer, Acrylates/Steareth-20 Methacrylate Crosspolymer, Polyacrylate Crosspolymer-6, and mixtures thereof, and wherein the microcapsules have an average particle size as median volume particle size D(v;0.5) value comprised between about 5 and 30 microns.

2. The composition according to claim 1, wherein the surfactant is present in an amount comprised between about 5% and about 45% by weight over the weight of the composition.

3. The composition according to claim 1 or claim 2, wherein the surfactant is selected from the group consisting of:
(S1) an alkyl benzene sulfonate having formula (i) R-Ar-SO₃M wherein R is a C₃-C₂₄ alkyl group, Ar is a phenyl group, and M is a cosmetically acceptable cation;
(S2) an alkyl sulfate having formula (ii) R-OSO₃M, wherein R and M are each independently as defined as above;
(S3) an alkyl ether sulfate having formula (iii) R-O(C₂H₄O)ₓSO₃M, wherein R and M are each independently as defined above and x is an integer comprised between 1 and 10;
(S4) an olefin sulfonate having formula (iv) L-SO₃M wherein L is a C₁₀-C₂₄ alkenyl group and M is as defined above;
(S5) a sulfosuccinate;
(S6) a surfactant selected from the group consisting of cocoamphoacetate, cocoamphodiacetate, lauroamphoacetate, lauroamphodiacetate, and mixtures thereof; and
(S7) mixtures thereof.

4. The composition according to any one of claims 1 to 3, wherein the suspending agent is present in an amount comprised about 0.01 and about 5.0 wt.% over the weight of the composition.

5. The composition according to any one of claims 1 to 4, further comprising a polymer deposition aid.

6. The composition according to claim 5, wherein the deposition aid is a cationic one.

7. The composition according to claim 6, wherein the deposition aid includes a polymer containing quaternary amine groups.

8. The composition according to claim 6 or claim 7, wherein the cationic deposition aid has an average molecular weight comprised between about 250,000 and about 3 million amu and a charge density comprised between about 1.0 and about 4 meq/g at a pH comprised between about 3 and 9.

9. The composition according to claim 5, wherein the deposition aid includes a cationic polymer derivative of natural polysaccharide polymers.

10. The composition according to claim 9, wherein the cationic polymer derivative is a polymer having a C₅-C₆ sugar backbone grafted with cationic functional moieties.

11. The composition according to any one of claims 5 to 10, wherein the deposition aid is present in an amount comprised between about 0.01 wt.% and about 5.0 wt.% by weight over the weight of the composition.

12. The composition according to any one of claims 1 to 11, further comprising a salt.

13. The composition according to any one of claims 1 to 12, wherein the carrier includes water in an amount such that the composition contains between about 50% and 90% by weight of water.

14. The composition according to any one of claims 1 to 13, wherein the microcapsules have the structure of a perfume composition enclosed within a polymeric shell.

15. The composition according to claim 14, wherein the microcapsules have a density higher than about 0.85 and lower than about 1.1 g/ml as measured at 20 degrees and 1 atm.

16. The composition according to claim 14 or claim 15, wherein the microcapsules have a polymeric shell which is obtainable by any one of:
A) condensation reactions,
B) free radical polymerization reactions, or
C) coacervation of pre-formed polymers followed by crosslinking of the thereby obtained coacervates by using a crosslinker.

17. The composition according to any one of claims 1 to 16, having a viscosity of lower than about 40000 cPs and greater than about 500 cPs when measured at 20 degrees and 1 atm by using a Brookfield viscometer at 20 or lower rpm.

18. Use of an ingredient selected from the group consisting of (INCI names) Acrylates/C10-30Alkyl Acrylate Crosspolymer, Acrylates/Steareth-20 Methacrylate Crosspolymer, Polyacrylate Crosspolymer-6, and mixtures thereof, as suspending agent in a liquid rinse-off cosmetic composition, said composition comprising an aqueous carrier and a plurality of perfume-containing, formaldehyde-free microcapsules dispersed therein, wherein the microcapsules have an average particle size as median volume particle size D(v;0.5) value comprised between about 5 and 30 microns.

## Patentansprüche

1. Abzuspülende kosmetische Zusammensetzung, umfassend einen wässrigen Träger und mehrere parfumhaltige, formaldehydfreie Mikrokapseln, die in der Zusammensetzung dispergiert sind, wobei die Zusammensetzung flüssig ist, wobei die Zusammensetzung ein Tensid und ein Suspensionsmittel umfasst, das aus der aus Acrylat/C10-30-Alkylacrylat-Crosspolymer, Acrylat/Steareth-20-Methacrylat-Crosspolymer, Polyacrylat-Crosspolymer-6 und Mischungen davon bestehenden Gruppe ausgewählt ist, und wobei die Mikrokapseln als einen Medianvolumenpartikelgrößen-D(v;0,5)-Wert eine durchschnittliche Partikelgröße von ungefähr 5 bis 30 Mikron aufweisen.

2. Zusammensetzung nach Anspruch 1, wobei das Tensid in einer Menge von ungefähr 5 bis 45 Gewichts-% in Bezug auf das Gewicht der Zusammensetzung vorhanden ist.

3. Zusammensetzung nach Anspruch 1 oder 2, wobei das Tensid aus der Gruppe ausgewählt ist, die besteht aus:
(S1) einem Alkylbenzolsulfonat mit der Formel (i) R-Ar-SO₃M, wobei R eine C₃-C₂₄-Alkylgruppe ist, Ar eine Phenylgruppe ist und M ein kosmetisch akzeptables Kation ist,
(S2) einem Alkylsulfat mit der Formel (ii) R-OSO₃M, wobei R und M jeweils unabhängig wie oben definiert sind,
(S3) einem Alkylethersulfat mit der Formel (iii) R-O(C₂H₄O)ₓSO₃M, wobei R und M jeweils unabhängig wie oben definiert sind und x eine ganze Zahl von 1 bis 10 ist,
(S4) einem Olefinsulfonat mit der Formel (iv) L-SO₃M, wobei L eine C₁₀-C₂₄-Alkenylgruppe ist und M wie oben definiert ist,
(S5) einem Sulfosuccinat,
(S6) einem Tensid, das aus der aus Cocoamphoacetat, Cocoamphodiacetat, Lauroamphoacetat, Lauroamphodiacetat und Mischungen davon bestehenden Gruppe ausgewählt ist, und
(S7) Mischungen davon.

4. Zusammensetzung nach einem der Ansprüche 1 bis 3, wobei das Suspensionsmittel in einer Menge von ungefähr 0,01 bis 5,0 Gew.-% in Bezug auf das Gewicht der Zusammensetzung vorhanden ist.

5. Zusammensetzung nach einem der Ansprüche 1 bis 4, ferner umfassend eine Polymerabscheidungshilfe.

6. Zusammensetzung nach Anspruch 5, wobei die Abscheidungshilfe eine kationische ist.

7. Zusammensetzung nach Anspruch 6, wobei die Abscheidungshilfe ein Polymer umfasst, das quaternäre Amingruppen enthält.

8. Zusammensetzung nach Anspruch 6 oder 7, wobei die kationische Abscheidungshilfe ein durchschnittliches Molekulargewicht von ungefähr 250.000 bis ungefähr 3 Millionen amu und eine Ladungsdichte von ungefähr 1,0 bis 4 meq/g bei einem pH-Wert von ungefähr 3 bis 9 aufweist.

9. Zusammensetzung nach Anspruch 5, wobei die Abscheidungshilfe ein kationisches Polymerderivat natürlicher Polysaccharidpolymere umfasst.

10. Zusammensetzung nach Anspruch 9, wobei das kationische Polymerderivat ein Polymer ist, das ein C₅-C₆-Zucker-Rückgrat aufweist und das mit kationischen funktionellen Resten gepfropft ist.

11. Zusammensetzung nach einem der Ansprüche 5 bis 10, wobei die Abscheidungshilfe in einer Menge von ungefähr 0,01 bis 5,0 Gew.-% in Bezug auf das Gewicht der Zusammensetzung vorhanden ist.

12. Zusammensetzung nach einem der Ansprüche 1 bis 11, ferner umfassend ein Salz.

13. Zusammensetzung nach einem der Ansprüche 1 bis 12, wobei der Träger Wasser in einer solchen Menge umfasst, dass die Zusammensetzung ungefähr 50 bis 90 Gewichts-% Wasser enthält.

14. Zusammensetzung nach einem der Ansprüche 1 bis 13, wobei die Mikrokapseln die Struktur aufweisen, bei der eine Parfumzusammensetzung in einer polymeren Hülle eingekapselt ist.

15. Zusammensetzung nach Anspruch 14, wobei die Mikrokapseln eine Dichte aufweisen, die mehr als ungefähr 0,85 und weniger als ungefähr 1,1 g/ml beträgt, gemessen bei 20 Grad und 1 atm.

16. Zusammensetzung nach Anspruch 14 oder 15, wobei die Mikrokapseln eine polymere Hülle aufweisen, die durch eine der folgenden erhältlich ist:
A) Kondensationsreaktionen,
B) freie Radikalpolymerisationsreaktionen oder
C) Koazervation vorgeformter Polymere, gefolgt von Quervernetzen der dadurch erhaltenen Koazervate unter Verwendung eines Quervernetzers.

17. Zusammensetzung nach einem der Ansprüche 1 bis 16, die eine Viskosität von weniger als ungefähr 40.000 cPs und mehr als ungefähr 500 cPs aufweist, wenn diese bei 20 Grad und 1 atm unter Verwendung eines Brookfield-Viskosimeters bei 20 oder weniger Upm gemessen wird.

18. Verwendung eines Inhaltsstoffs, der aus der aus (INCI Namen) Acrylat/C10-30-Alkylacrylat-Crosspolymer, Acrylat/Steareth-20-Methacrylat-Crosspolymer, Polyacrylat-Crosspolymer-6 und Mischungen davon bestehenden Gruppe ausgewählt ist, als Suspensionsmittel in einer flüssigen abzuspülenden kosmetischen Zusammensetzung, wobei die Zusammensetzung einen wässrigen Träger und mehrere parfumhaltige, formaldehydfreie Mikrokapseln, die darin dispergiert sind, umfasst, wobei die Mikrokapseln als einen Medianvolumenpartikelgrößen-D(v;0,5)-Wert eine durchschnittliche Partikelgröße von ungefähr 5 bis 30 Mikron aufweisen.

## Revendications

1. Composition cosmétique à rincer comprenant un véhicule aqueux et une pluralité de microcapsules exemptes de formaldéhyde et contenant du parfum, dispersées dans la composition, où la composition est liquide, la composition comprend un tensioactif et un agent de suspension choisi dans le groupe constitué de : Acrylates/C10-30Alkyl Acrylate Crosspolymer, Acrylates/Steareth-20 Crosspolymer, Polyacrylate Crosspolymer-6, et les mélanges de ceux-ci, et où les microcapsules ont une taille moyenne de particules, exprimées comme taille moyenne de particules en volume D(v;0.5) comprise entre environ 5 et environ 30 microns.

2. Composition selon la revendication 1, dans laquelle le tensioactif est présent en une quantité comprise entre environ 5% et environ 45% en masse, par rapport à la masse de la composition.

3. Composition selon la revendication 1 ou la revendication 2, dans laquelle le tensioactif est choisi dans le groupe constitué de :
(S1) un benzènesulfonate d'alkyle de formule (i) R-Ar-SO₃M dans laquelle R est un groupe C₃-C₂₄ alkyle, Ar est un groupe phényle, et M est un cation cosmétiquement acceptable ;
(S2) un sulfate d'alkyle de formule (ii) R-OSO₃M, dans laquelle R et M sont chacun indépendamment tels que définis ci-dessus ;
(S3) un éther sulfate d'alkyle de formule (iii) R-O(C₂H₄O)ₓSO₃M, dans laquelle R et M sont chacun indépendamment tels que définis ci-dessus et x est un entier allant de 1 à 10;
(S4) un sulfonate d'oléfine de formule (iv) L-SO₃M dans laquelle L est un groupe C₁₀-C₂₄ alcényle et M est tel que défini ci-dessus ;
(S5) un sulfosuccinate ;
(S6) un tensioactif choisi dans le groupe constitué de : cocoamphoacétate, cocoamphodiacétate, lauroamphoacétate, lauroamphodiacétate, et les mélanges de ceux-ci ; et
(S7) les mélanges de ces tensioactifs.

4. Composition selon l'une quelconque des revendications 1 à 3, dans laquelle l'agent de suspension est présent en une quantité comprise entre environ 0,01 et environ 5,0% en masse par rapport à la masse de la composition.

5. Composition selon l'une quelconque des revendications 1 à 4, qui comprend en outre une aide polymérique au dépôt.

6. Composition selon la revendication 5, dans laquelle l'aide au dépôt est une aide cationique.

7. Composition selon la revendication 6, dans laquelle l'aide au dépôt inclut un polymère contenant des groupes amine quaternaire.

8. Composition selon la revendication 6 ou la revendication 7, dans laquelle l'aide au dépôt cationique a une masse moléculaire moyenne comprise entre environ 250,000 et environ 3 millions amu et une densité de charge comprise entre environ 1,0 et environ 4 meq/g à un pH compris entre environ 3 et 9.

9. Composition selon la revendication 5, dans laquelle l'aide au dépôt inclut un polymère cationique dérivé de polymère polysaccharidique naturel.

10. Composition selon la revendication 9, dans laquelle le dérivé de polymère cationique est un polymère ayant le squelette d'un sucre en C₅-C₆ greffé avec des groupes fonctionnels cationiques.

11. Composition selon l'une quelconque des revendications 5 à 10, dans laquelle l'aide au dépôt est présente en une quantité comprise entre environ 0,01 % en masse et environ 5,0 % en masse par rapport à la masse de la composition.

12. Composition selon l'une quelconque des revendications 1 à 11, qui comprend en outre un sel.

13. Composition selon l'une quelconque des revendications 1 à 12, dans laquelle le véhicule inclut de l'eau en une quantité telle que la composition contient entre environ 50% et 90% en masse d'eau.

14. Composition selon l'une quelconque des revendications 1 à 13, dans laquelle les microcapsules ont la structure d'une composition de parfum encapsulée dans une enveloppe polymérique.

15. Composition selon la revendication 14, dans laquelle les microcapsules ont une densité supérieure à environ 0,85 et inférieure à environ 1,1 g/ml telle que mesurée à 20 degrés et sous 1 atm.

16. Composition selon la revendication 14 ou la revendication 15, dans laquelle les microcapsules ont une enveloppe polymérique susceptible d'être obtenue par l'une quelconque de :
A) réaction de condensation ;
B) réaction de polymérisation radicalaire libre ; ou
C) coacervation de polymères préformés suivi de réticulation des coacervats ainsi obtenus à l'aide d'un réticulant.

17. Composition selon l'une quelconque des revendications 1 à 16, qui a une viscosité inférieure à environ 40000 cPs et supérieure à environ 500 cPs lorsqu'elle est mesurée à 20 degrés et sous 1 atm à l'aide d'un viscosimètre Brookfield à une vitesse inférieure ou égale à 20 tr/min.

18. Utilisation d'un ingrédient choisi dans le groupe constitué de (noms INCI): Acrylates/C10-30Alkyl Acrylate Crosspolymer, Acrylates/Steareth-20 Crosspolymer, Polyacrylate Crosspolymer-6, et les mélanges de ceux-ci, comme agent de suspension dans une composition cosmétique à rincer, ladite composition comprenant un véhicule aqueux et une pluralité de microcapsules exemptes de formaldéhyde et contenant du parfum, dispersées dans la composition, où les microcapsules ont une taille moyenne de particules, exprimées comme taille moyenne de particules en volume D(v;0.5) comprise entre environ 5 et environ 30 microns.
